# EUROPÄISCHE PATENTSCHRIFT

(11) **EP 1 480 516 B1**
(45) Veröffentlichungstag und Bekanntmachung des Hinweises auf die Patenterteilung: **03.02.2010**
(21) Anmeldenummer: 03742514.7
(22) Anmeldetag: 11.02.2003
(51) Int. Cl.: A01N 37/22

(54) **MIKROBIZIDE MITTEL AUF BASIS VON BIPHENYLBENZAMID-DERIVATEN**
MICROBICIDAL AGENTS ON THE BASIS OF BIPHENYL BENZAMIDE DERIVATIVES
AGENTS MICROBICIDES A BASE DE DERIVES DE BIPHENYLE BENZAMIDE

(30) Priorität: 23.02.2002 DE 10207773; 08.04.2002 DE 10215291
(43) Veröffentlichungstag der Anmeldung: 01.12.2004
(73) Patentinhaber: Bayer CropScience AG, 40789 Monheim (DE)
(72) Erfinder: RIECK, Heiko, F-69110 Ste. Foy-lès-Lyon (FR); DUNKEL, Ralf, 40789 Monheim (DE); ELBE, Hans-Ludwig, 42329 Wuppertal (DE); WACHENDORFF-NEUMANN, Ulrike, 56566 Neuwied (DE); MAULER-MACHNIK, Astrid, 42799 Leichlingen (DE); KUCK, Karl-Heinz, 40764 Langenfeld (DE)
(86) Internationale Anmeldenummer: PCT/EP2003/001322
(87) Internationale Veröffentlichungsnummer: WO 2003/069995

(56) Entgegenhaltungen:
- EP-A- 0 545 099
- DATABASE CA [Online] CHEMICAL ABSTRACTS SERVICE, COLUMBUS, OHIO, US; WHITE, G. A.: "Substituted benzanilides: structural variation and inhibition of complex II activity in mitochondria from a wild-type strain and a carboxin-selected mutant strain of Ustilago maydis" retrieved from STN Database accession no. 106:191006 XP002239592 & PESTICIDE BIOCHEMISTRY AND PHYSIOLOGY (1987), 27(3), 249-60 ,

## Beschreibung

Die vorliegende Erfindung betrifft neue mikrobizide Mittel auf Basis von teilweise bekannten Biphenylbenzamid-Derivaten und die Verwendung dieser Stoffe zur Bekämpfung von unerwünschten Mikroorganismen. Außerdem betrifft die Erfindung auch neue Biphenylbenzamid-Derivaten und mehrere Verfahren zu deren Herstellung.

Es ist bereits bekannt, dass Biphenylbenzamid-Derivate zur Bekämpfung von Arten des phytopathogenen Pilzes Botrytis eingesetzt werden können (vgl. EP-A 0 545 099). Die Wirksamkeit dieser vorbekannten Verbindungen ist jedoch insbesondere bei niedrigen Aufwandmengen nicht in allen Anwendungsgebieten völlig zufriedenstellend.

Weiterhin sind bestimmte Biphenylbenzamid-Derivate bekannt, wie beispielsweise die Verbindungen *N-*(2'-Fluor-1,1'-biphenyl-2-yl)-2-(trifluormethyl)benzamid und *N*-(4'-Fluor-1,1'-biphenyl-2-yl)-2-(trifluormethyl)benzamid (vgl. EP-A 0 545 099).

Über eine breite fungizide Einsetzbarkeit dieser Verbindungen ist bisher nichts bekannt. Weiterhin ist nicht bekannt, inwieweit diese Verbindungen gegen andere mikrobielle, z.B. bakterielle, Schädlinge eingesetzt werden können.

Es wurde nun gefunden, dass die Biphenylbenzamid-Derivate der Formel (I) 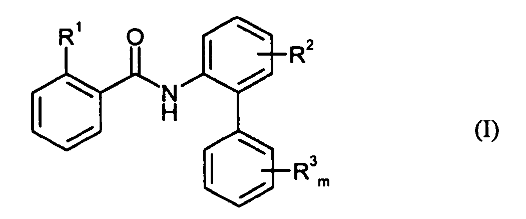 in welcher
- R¹: für Methyl, Trifluormethyl, Chlor, Brom oder Iod steht,
- R²: für Wasserstoff oder Fluor steht,
- R³: für Halogen, Cyano, Nitro, C₁-C₆-Alkyl, C₁-C₄-Alkoxy, C₁-C₄-Alkylthio, C₁-C₄-Alkylsulfonyl, C₂-C₆-Alkenyl, C₃-C₆-Cycloalkyl, oder für C₁-C₆-Halogenalkyl, C₁-C₆-Halogenalkoxy, C₁-C₆-Halogenalkylthio oder C₁-C₆-Halogenalkylsulfonyl mit jeweils 1 bis 13 Halogenatomen steht,
- m: für 1, 2, 3, 4 oder 5 steht, wobei die Reste R³ gleich oder verschieden sein können,
sehr gut zur Bekämpfung von phytopathogenen Schaderregern aus der Klasse der Chytridiomycetes (Unterabteilung der Mastigomycotina), der Klasse der Zygomycetes (Zygomycotina), der Klassen der Hemiascomycetes, Plectomycetes, Pyrenomycetes, Laboulbeniomycetes, Locoloascomycetes (Ascomycotina), sowie aus den Unterabteilungen der Basidiomycotina und der Deuteromycotina, sowie schädlicher Mikroorganismen im Materialschutz geeignet sind.

Die Verbindungen der Formel (I) können in Abhängigkeit von der Art der Substituenten als geometrische und/oder optische Isomere oder Isomerengemische unterschiedlicher Zusammensetzung vorliegen. Die Erfindung betrifft sowohl die Verwendung der reinen Isomeren als auch der Isomerengemische.

Die erfindungsgemäß verwendbaren Biphenylbenzamid-Derivate sind durch die Formel (I) allgemein definiert.

Bevorzugt verwendbar sind Biphenylbenzamid-Derivate der Formel (I), in welcher
- R¹: für Trifluormethyl, Chlor, Brom oder Iod steht,
- R²: für Wasserstoff oder Fluor steht,
- R³: für Halogen, C₁-C₄-Alkyl, C₁-C₄-Alkoxy, C₁-C₄-Alkylthio, C₂-C₄-Alkenyl, Cyclopropyl, Cyclobutyl, Cyclopentyl, Cyclohexyl, oder für C₁-C₄-Halogenalkyl, C₁-C₄-Halogenalkoxy oder C₁-C₄-Halogenalkylthio mit jeweils 1 bis 9 Halogenatomen steht,
- m: für 1, 2, 3 steht, wobei die Reste R³ gleich oder verschieden sein können.

Besonders bevorzugt verwendbar sind Biphenylbenzamid-Derivate der Formel (I), in welcher
- R¹: für Trifluormethyl oder Iod steht,
- R²: für Wasserstoff steht,
- R³: für Fluor, Chlor, Brom, Iod, Methyl, Ethyl, n-, i-Propyl, n-, i-, s-, t-Butyl, Methoxy, Ethoxy, Methylthio, Ethylthio, oder für C₁ -C₂-Halogenalkyl, C₁-C₂-Halogenalkoxy oder C₁-C₂-Halogenalkylthio mit jeweils 1 bis 5 Halogenatomen steht,
- m: für 1, 2 steht, wobei die Reste R³ gleich oder verschieden sein können,

Ganz besonders bevorzugt verwendbar sind Biphenylbenzamid-Derivate der Formel (I), in welcher
- R¹: für Trifluormethyl oder Iod steht,
- R²: für Wasserstoff steht,
- R³: für Fluor, Chlor, Brom, Methyl, Methoxy, Methylthio, Trifluormethyl, Trifluormethoxy oder Trifluormethylthio steht,
- m: für 1, 2 steht, wobei die Reste R³ gleich oder verschieden sein können.

Weiterhin ganz besonders bevorzugt verwendbar sind Verbindungen der Formel (I), in welcher R¹ für Trifluormethyl steht.
Weiterhin ganz besonders bevorzugt verwendbar sind Verbindungen der Formel (I), in welcher R¹für Iod steht.
Weiterhin ganz besonders bevorzugt verwendbar sind Verbindungen der Formel (I), in welcher R² für Wasserstoff steht.
Weiterhin ganz besonders bevorzugt verwendbar sind Verbindungen der Formel (I), in welcher R² für Fluor steht.
Weiterhin ganz besonders bevorzugt verwendbar sind Verbindungen der Formel (I), in welcher R³ für Fluor, Chlor oder Brom steht.
Weiterhin ganz besonders bevorzugt verwendbar sind Verbindungen der Formel (I), in welcher R³ für Trifluormethyl, Trifluormethoxy oder Trifluormethylthio steht.
Weiterhin ganz besonders bevorzugt verwendbar sind Verbindungen der Formel (I), in welcher m für 2 steht.

Die erfindungsgemäß verwendbaren Biphenylbenzamid-Derivate der Formel (I) eignen sich sehr gut zur Bekämpfung von phytopathogenen Schaderregern der Klassen Chytridiomycetes, Zygomycetes, Hemiascomycetes, Plectomycetes, Pyrenomycetes, Laboulbeniomycetes, Locoloascomycetes, Basidiomycetes und Deuteromycetes, sowie schädlicher Mikroorganismen im Materialschutz wie Pseudomonadaceae, Rhizobiaceae, Enterobacteriaceae, Corynebacteriaceae und Streptomycetaceae.

Beispielhaft aber nicht begrenzend seien einige Erreger von pilzlichen und bakteriellen Erkrankungen, die unter die oben aufgezählten Oberbegriffe fallen, genannt:
Xanthomonas-Arten, wie beispielsweise Xanthomonas campestris pv. oryzae;
Pseudomonas-Arten, wie beispielsweise Pseudomonas syringae pv. lachrymans;
Erwinia-Arten, wie beispielsweise Erwinia amylovora;
Erysiphe-Arten, wie beispielsweise Erysiphe graminis;
Sphaerotheca-Arten, wie beispielsweise Sphaerotheca fuliginea;
Podosphaera-Arten, wie beispielsweise Podosphaera leucotricha;
Venturia-Arten, wie beispielsweise Venturia inaequalis;
Pyrenophora-Arten, wie beispielsweise Pyrenophora teres oder P. graminea (Konidienform: Drechslera, Syn: Helminthosporium);
Cochliobolus-Arten, wie beispielsweise Cochliobolus sativus (Konidienform: Drechslera, Syn: Helminthosporium);
Uromyces-Arten, wie beispielsweise Uromyces appendiculatus;
Puccinia-Arten, wie beispielsweise Puccinia recondita;
Tilletia-Arten, wie beispielsweise Tilletia caries;
Ustilago-Arten, wie beispielsweise Ustilago nuda oder Ustilago avenae;
Pellicularia-Arten, wie beispielsweise Pellicularia sasakii;
Pyricularia-Arten, wie beispielsweise Pyricularia oryzae;
Fusarium-Arten, wie beispielsweise Fusarium culmorum;
Septoria-Arten, wie beispielsweise Septoria nodorum;
Leptosphaeria-Arten, wie beispielsweise Leptosphaeria nodorum;
Cercospora-Arten, wie beispielsweise Cercospora canescens;
Altemaria-Arten, wie beispielsweise Altemaria brassicae;
Pseudocercosporella-Arten, wie beispielsweise Pseudocercosporella herpotrichoides.

Bevorzugt lassen sich erfindungsgemäß folgende Erreger von pilzlichen und bakteriellen Erkrankungen durch Verwendung der Verbindungen der Formel (I) bekämpfen:
Pseudomonas-Arten, wie beispielsweise Pseudomonas syringae pv. lachrymans;
Erysiphe-Arten, wie beispielsweise Erysiphe graminis;
Sphaerotheca-Arten, wie beispielsweise Sphaerotheca fuliginea;
Podosphaera-Arten, wie beispielsweise Podosphaera leucotricha;
Venturia-Arten, wie beispielsweise Venturia inaequalis;
Pyrenophora-Arten, wie beispielsweise Pyrenophora teres oder P. graminea (Konidienform: Drechslera, Syn: Helminthosporium);
Cochliobolus-Arten, wie beispielsweise Cochliobolus sativus (Konidienform: Drechslera, Syn: Helminthosporium);
Uromyces-Arten, wie beispielsweise Uromyces appendiculatus;
Puccinia-Arten, wie beispielsweise Puccinia recondita;
Tilletia-Arten, wie beispielsweise Tilletia caries;
Ustilago-Arten, wie beispielsweise Ustilago nuda oder Ustilago avenae;
Pellicularia-Arten, wie beispielsweise Pellicularia sasakii;
Pyricularia-Arten, wie beispielsweise Pyricularia oryzae;
Septoria-Arten, wie beispielsweise Septoria nodorum;
Leptosphaeria-Arten, wie beispielsweise Leptosphaeria nodorum;
Cercospora-Arten, wie beispielsweise Cercospora canescens;
Alternaria-Arten, wie beispielsweise Altemaria brassicae;
Pseudocercosporella-Arten, wie beispielsweise Pseudocercosporella herpotrichoides.

Besonders bevorzugt, lassen sich erfindungsgemäß folgende Erreger von pilzlichen und bakteriellen Erkrankungen durch Verwendung der Verbindungen der Formel (I) bekämpfen:
Sphaerotheca-Arten, wie beispielsweise Sphaerotheca fuliginea;
Podosphaera-Arten, wie beispielsweise Podosphaera leucotricha;
Venturia-Arten, wie beispielsweise Venturia inaequalis;
Uromyces-Arten, wie beispielsweise Uromyces appendiculatus;
Puccinia-Arten, wie beispielsweise Puccinia recondita;
Pellicularia-Arten, wie beispielsweise Pellicularia sasakii;
Cercospora-Arten, wie beispielsweise Cercospora canescens;
Alternaria-Arten, wie beispielsweise Altemaria brassicae.

Biphenylbenzamid-Derivate der Formel (I) sind aus EP-A 0 545 099 bekannt.

Neu sind die Biphenylbenzamid-Derivate der Formel (I) (Gruppe 1) 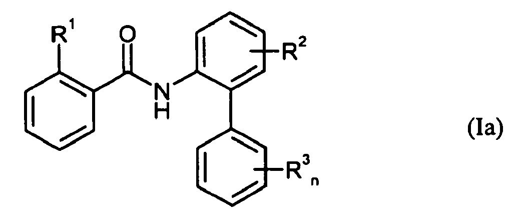 in welcher
- R¹: für Methyl, Trifluormethyl, Chlor, Brom oder Iod steht,
- R²: für Wasserstoff oder Fluor steht,
- R³: für Halogen, Cyano, Nitro, C₁-C₆-Alkyl, C₁-C₄-Alkoxy, C₁-C₄-Alkylthio, C₁-C₄-Alkylsulfonyl, C₂-C₆-Alkenyl, C₃-C₆-Cycloalkyl, oder für C₁-C₆-Halogenalkyl, C₁-C₆-Halogenalkoxy, C₁-C₆-Halogenalkylthio oder C₁-C₆-Halogenalkylsulfonyl mit jeweils 1 bis 13 Halogenatomen steht,
- n: für 2, 3, 4 oder 5 steht, wobei die Reste R³ gleich oder verschieden sein können.

Bevorzugt sind Biphenylbenzamid-Derivate der Formel (I), in welcher
- R¹: für Trifluormethyl, Chlor, Brom oder Iod steht,
- R²: für Wasserstoff oder Fluor steht,
- R³: für Halogen, C₁-C₄-Alkyl, C₁-C₄-Alkoxy, C₁-C₄-Alkylthio, C₂-C₄-Alkenyl, Cyclopropyl, Cyclobutyl, Cyclopentyl, Cyclohexyl, oder für C₁-C₄-Halogenalkyl, C₁-C₄-Halogenalkoxy oder C₁-C₄-Halogenalkylthio mit jeweils 1 bis 9 Halogenatomen steht,
- n: für 2, 3 steht, wobei die Reste R³ gleich oder verschieden sein können.

Besonders bevorzugt sind Biphenylbenzamid-Derivate der Formel (I), in welcher
- R¹: für Trifluormethyl oder Iod steht,
- R²: für Wasserstoff steht,
- R³: für Fluor, Chlor, Brom, Iod, Methyl, Ethyl, n-, i-Propyl, n-, i-, s-, t-Butyl, Methoxy, Ethoxy, Methylthio, Ethylthio, oder für C₁-C₂-Halogenalkyl, C₁-C₂-Halogenalkoxy oder C₁-C₂-Halogenalkylthio mit jeweils 1 bis 5 Halogenatomen steht,
- n: für 2 steht, wobei die Reste R³ gleich oder verschieden sein können.

Ganz besonders bevorzugt sind Biphenylbenzamid-Derivate der Formel (I), in welcher
- R¹: für Trifluormethyl oder Iod steht,
- R²: für Wasserstoff steht,
- R³: für Fluor, Chlor, Brom, Methyl, Methoxy, Methylthio, Trifluormethyl, Trifluormethoxy oder Trifluormethylthio steht,
- n: für 2 steht, wobei die Reste R³ gleich oder verschieden sein können.

Weiterhin wurde gefunden, dass die neuen Biphenylbenzamid-Derivate der Formel (I) sehr gute mikrobizide Eigenschaften besitzen und zur Bekämpfung unerwünschter Mikroorganismen sowohl im Pflanzenschutz als auch im Materialschutz verwendbar sind.

Die oben aufgeführten allgemeinen oder in Vorzugsbereichen aufgeführten Restedefinitionen bzw. Erläuterungen können auch untereinander, also zwischen den jeweiligen Bereichen und Vorzugsbereichen beliebig kombiniert werden. Sie gelten für die Endprodukte sowie für die Vor- und Zwischenprodukte entsprechend. Außerdem können auch einzelne Definitionen entfallen.

Gesättigte Kohlenwasserstoffreste wie Alkyl können, auch in Verbindung mit Heteroatomen, wie z.B. in Alkoxy, soweit möglich, jeweils geradkettig oder verzweigt sein.
Durch Halogen substituierte Reste, z.B. Halogenalkyl, sind einfach oder mehrfach bis zur maximal möglichen Substituentenzahl halogeniert. Bei mehrfacher Halogenierung können die Halogenatome gleich oder verschieden sein. Halogen steht dabei für Fluor, Chlor, Brom oder Iod, insbesondere für Fluor, Chlor oder Brom.

Biphenylbenzamid-Derivate der Formel (Ia), sind jeweils Untergruppen der erfindungsgemäß verwendbaren Verbindungen der Formel (I). Diese Verbindungen lassen sich auf grundsätzlich gleiche Weise herstellen. Daher wird im folgenden exemplarisch die Herstellung der Verbindungen der Formel (I) beschrieben. Verbindungen der Formel (I) lassen sich herstellen, indem man
A) Benzoylhalogenide der Formel (II) 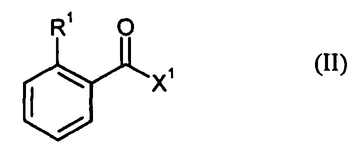 in welcher
   - R¹: die oben angegebenen Bedeutungen hat,
   - X¹: für Halogen steht,
   mit Anilinderivaten der Formel (III) 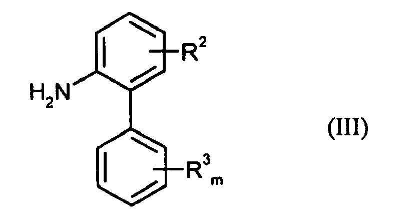 in welcher
   - R², R³ und m: die oben angegebenen Bedeutungen haben,
   gegebenenfalls in Gegenwart eines Säurebindemittels und gegebenenfalls in Gegenwart eines Verdünnungsmittels umsetzt, oder
B) Halogenbenzamide der Formel (IV) 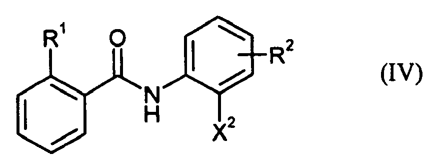 in welcher
   - R¹ und R²: die oben angegebenen Bedeutungen haben,
   - X²: für Brom oder Iod steht,
   mit Boronsäurederivaten der Formel (V) 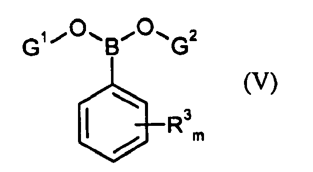 in welcher
   - R³ und m: die oben angegebenen Bedeutungen haben,
   - G¹ und G²: jeweils für Wasserstoff oder zusammen für Tetramethylethylen stehen,
   in Gegenwart eines Katalysators, gegebenenfalls in Gegenwart eines Säurebindemittels und gegebenenfalls in Gegenwart eines Verdünnungsmittels umsetzt, oder
C) Benzamid-Boronsäure-Derivate der Formel (VI) 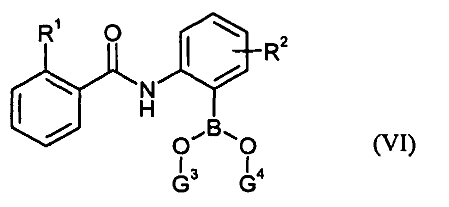 in welcher
   - R¹ und R²: die oben angegebenen Bedeutungen haben,
   - G³ und G⁴: jeweils für Wasserstoff oder zusammen für Tetramethylethylen stehen,
   mit Halogenbenzolderivaten der Formel (VII) 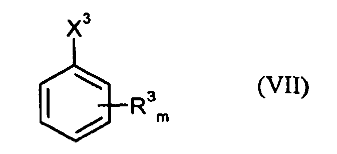 in welcher
   - R³ und m: die oben angegebenen Bedeutungen haben und
   - X³: für Brom, Iod oder Trifluormethylsulfonyloxy steht,
   in Gegenwart eines Katalysators, gegebenenfalls in Gegenwart eines Säurebindemittels und gegebenenfalls in Gegenwart eines Verdünnungsmittels umsetzt, oder
D) Halogenbenzamide der Formel (IV) 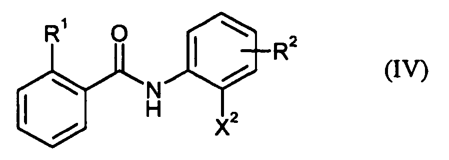 in welcher
   - R¹ und R²: die oben angegebenen Bedeutungen haben,
   - X²: für Brom oder Iod steht,
   in einer ersten Stufe mit einem Diboran-Derivat der Formel (VIII) 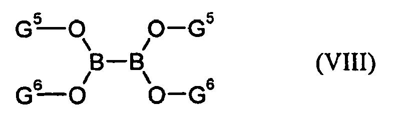 in welcher
   - G⁵ und G⁶: jeweils für Alkyl oder gemeinsam für Alkandiyl stehen,
   in Gegenwart eines Katalysators, gegebenenfalls in Gegenwart eines Säurebindemittels und gegebenenfalls in Gegenwart eines Verdünnungsmittels umsetzt und ohne Aufarbeitung in einer zweiten Stufe mit Halogenbenzolderivaten der Formel (VII) 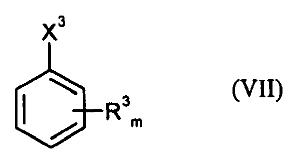 in welcher
   - R³ und m: die oben angegebenen Bedeutungen haben und
   - X³: für Brom, Iod oder Trifluormethylsulfonyloxy steht,
   in Gegenwart eines Katalysators, gegebenenfalls in Gegenwart eines Säurebindemittels und gegebenenfalls in Gegenwart eines Verdünnungsmittels umsetzt.

Verwendet man beispielsweise 2-(Trifluormethyl)benzoylchlorid und 4'-Chlor-1,1'-biphenyl-2-amin als Ausgangsstoffe sowie ein Base, so kann der Verlauf des erfindungsgemäßen Verfahrens A) durch folgende Reaktionsgleichung veranschaulicht werden:

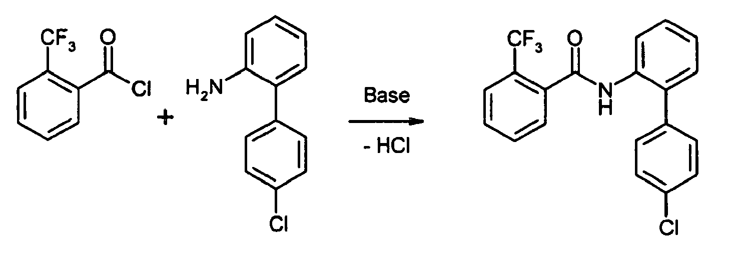

Die zur Durchführung des erfindungsgemäßen Verfahrens A) als Ausgangsstoffe benötigten Benzoylhalogenide sind durch die Formel (II) allgemein definiert. In dieser Formel (II) steht R¹ bevorzugt, besonders bevorzugt bzw. ganz besonders bevorzugt für diejenigen Bedeutungen, die bereits im Zusammenhang mit der Beschreibung der erfindungsgemäß verwendbaren Verbindungen der Formel (I) als bevorzugt, besonders bevorzugt usw. für diesen Rest angegeben wurden. X¹ steht bevorzugt für Chlor.

Die Benzoylhalogenide der Formel (II) sind bekannt und/oder lassen sich nach bekannten Verfahren herstellen (vergleiche z.B. EP-A 0 276 177).

Die weiterhin zur Durchführung des erfindungsgemäßen Verfahrens A) als Ausgangsstoffe benötigten Anilinderivate sind durch die Formel (III) allgemein definiert. In dieser Formel (III) stehen R², R³ und m bevorzugt, besonders bevorzugt bzw. ganz besonders bevorzugt für diejenigen Bedeutungen, die bereits im Zusammenhang mit der Beschreibung der erfindungsgemäß verwendbaren Verbindungen der Formel (I) als bevorzugt, besonders bevorzugt usw. für diese Reste angegeben wurden.

Die Anilinderivate der Formel (III) sind bekannt und/oder lassen sich nach bekannten Methoden herstellen (vgl. z.B. Bull. Korean Chem. Soc. 2000, 21, 165-166; Chem. Pharm. Bull. 1992, 40, 240-4; JP 9132567).

Verwendet man beispielsweise *N*-(2-Iodphenyl)2-(trifluormethyl)benzamid und 4-Chlorphenylboronsäure als Ausgangsstoffe sowie einen Katalystor und eine Base, so kann der Verlauf des erfindungsgemäßen Verfahrens B) kann durch folgende Reaktionsgleichung veranschaulicht werden:

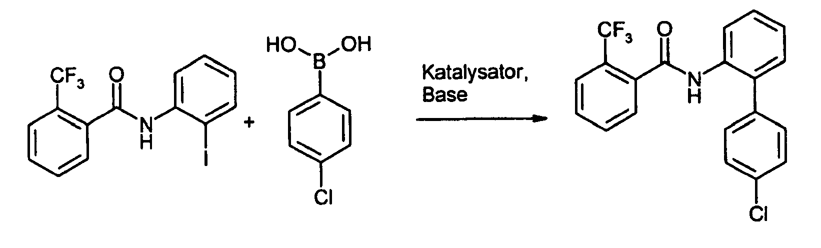

Die zur Durchführung des erfindungsgemäßen Verfahrens B) als Ausgangsstoffe benötigten Halogenbenzamide sind durch die Formel (IV) allgemein definiert. In dieser Formel (IV) stehen R¹ und R² bevorzugt, besonders bevorzugt bzw. ganz besonders bevorzugt für diejenigen Bedeutungen, die bereits im Zusammenhang mit der Beschreibung der erfindungsgemäß verwendbaren Verbindungen der Formel (I) als bevorzugt, besonders bevorzugt usw. für diese Reste angegeben wurden. X² steht bevorzugt für Brom oder Iod.

Die Halogenbenzamide der Formel (IV) sind noch nicht bekannt. Sie sind neue chemische Verbindungen und ebenfalls Gegenstand der vorliegenden Anmeldung. Sie werden erhalten, indem man
E) Benzoylhalogenide der Formel (II) 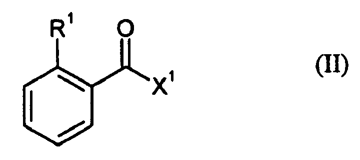 in welcher
   - R¹: die oben angegebenen Bedeutungen hat,
   - X¹: für Halogen steht,
   mit 2-Bromanilin oder 2-Iodanilin umsetzt.

Die zur Durchführung des erfindungsgemäßen Verfahrens E) als Ausgangsstoffe benötigten Benzoylhalogenide der Formel (II) sind bereits weiter oben im Zusammenhang mit dem erfindungsgemäßen Verfahren A) beschrieben worden.

Die weiterhin zur Durchführung des erfindungsgemäßen Verfahrens E) als Ausgangsstoffe benötigten Stoffe 2-Bromanilin oder 2-Iodanilin sind bekannte Synthesechemikalien.

Die weiterhin zur Durchführung des erfindungsgemäßen Verfahrens B) als Ausgangsstoffe benötigten Boronsäurederivate sind durch die Formel (V) allgemein definiert. In dieser Formel (V) stehen R³ und m bevorzugt, besonders bevorzugt bzw. ganz besonders bevorzugt für diejenigen Bedeutungen, die bereits im Zusammenhang mit der Beschreibung der erfindungsgemäß verwendbaren Verbindungen der Formel (I) als bevorzugt, besonders bevorzugt usw. für diese Reste angegeben wurden. G¹ und G² stehen bevorzugt jeweils für Wasserstoff oder zusammen für Tetramethylethylen.

Boronsäurederivate der Formel (V) sind bekannte Synthesechemikalien. Sie können auch unmittelbar vor der Reaktion direkt aus Halogenbenzolderivaten und Boronsäureestern hergestellt und ohne Aufarbeitung weiter umgesetzt werden.

Verwendet man beispielsweise *N-*[2-(4,4,5,5-Tetramethyl-1,3,2-dioxaborolan-2-yl)-phenyl]-2-(trifluormethyl)benzamid und 4-Chlorphenyl-trifluormethansulfonsäure als Ausgangsstoffe sowie einen Katalysator und eine Base, so kann der Verlauf des erfindungsgemäßen Verfahrens C) kann durch folgende Reaktionsgleichung veranschaulicht werden:

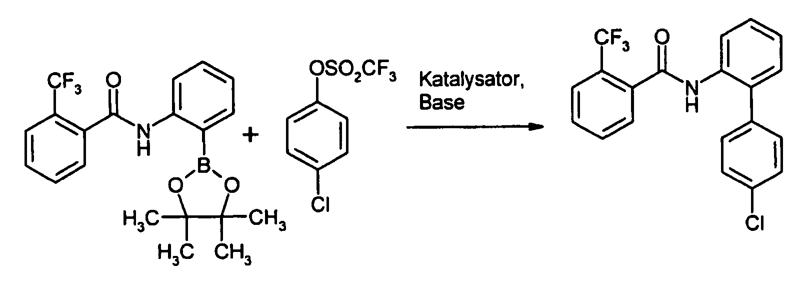

Die zur Durchführung des erfindungsgemäßen Verfahrens C) als Ausgangsstoffe benötigten Benzamid-Boronsäure-Derivate sind durch die Formel (VI) allgemein definiert. In dieser Formel (VI) stehen R¹ und R² bevorzugt, besonders bevorzugt bzw. ganz besonders bevorzugt für diejenigen Bedeutungen, die bereits im Zusammenhang mit der Beschreibung der erfindungsgemäß verwendbaren Verbindungen der Formel (I) als bevorzugt, besonders bevorzugt usw. für diese Reste angegeben wurden. G³ und G⁴ stehen bevorzugt jeweils für Wasserstoff oder zusammen für Tetramethylethylen.

Die Benzamid-Boronsäure-Derivate der Formel (VI) sind noch nicht bekannt. Sie sind neue chemische Verbindungen und ebenfalls Gegenstand der vorliegenden Anmeldung.

Sie werden erhalten, indem man
F) Benzoylhalogenide der Formel (II) 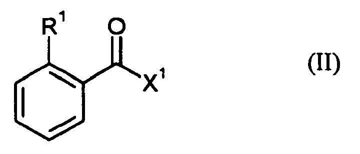 in welcher
   - R¹: die oben angegebenen Bedeutungen hat,
   - X¹: für Halogen steht,
   mit Anilinboronsäurederivaten der Formel (IX) 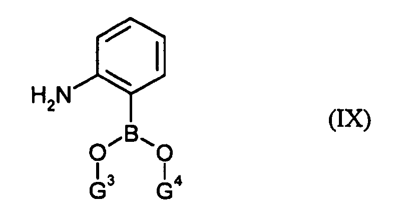 in welcher
   - G³ und G⁴: die oben angegebenen Bedeutungen haben,
   gegebenenfalls in Gegenwart eines Säurebindemittels und gegebenenfalls in Gegenwart eines Verdünnungsmittels umsetzt.

Die zur Durchführung des erfindungsgemäßen Verfahrens F) als Ausgangsstoffe benötigten Benzoylhalogenide der Formel (II) sind bereits weiter oben im Zusammenhang mit dem erfindungsgemäßen Verfahren A) beschrieben worden.

Die weiterhin zur Durchführung des erfindungsgemäßen Verfahrens F) als Ausgangsstoffe benötigten Anilinboronsäurederivate sind durch die Formel (IX) allgemein definiert. In dieser Formel (IX) stehen G³ und G⁴ bevorzugt jeweils für Wasserstoff oder zusammen für Tetramethylethylen.

Die zur Durchführung des erfindungsgemäßen Verfahrens F) als Ausgangsstoffe benötigten Anilinboronsäurederivate der Formel (IX) sind bekannte Synthesechemikalien.

Die weiterhin zur Durchführung des erfindungsgemäßen Verfahrens C) als Ausgangsstoffe benötigten Halogenbenzolderivate sind durch die Formel (VII) allgemein definiert. In dieser Formel (VII) stehen R³ und m bevorzugt, besonders bevorzugt bzw. ganz besonders bevorzugt für diejenigen Bedeutungen, die bereits im Zusammenhang mit der Beschreibung der erfindungsgemäß verwendbaren Verbindungen der Formel (I) als bevorzugt, besonders bevorzugt usw. für diese Reste angegeben wurden. X³ steht bevorzugt für Brom, Iod oder Trifluormethylsulfonyloxy.

Verwendet man beispielsweise *N*-(2-Bromphenyl)-2-(trifluormethyl)-benzamid und 4,4,4',4',5,5,5',5'-Octamethyl-2,2'-bi-1,3,2-dioxaborolan in der ersten Stufe und weiterhin 4-Brom-1-chlor-2-methylbenzol in der zweiten Stufe als Ausgangsstoffe, sowie in jeder Stufe einen Katalysator und eine Base, so kann der Verlauf des erfindungsgemäßen Verfahrens D) durch folgende Reaktionsgleichung veranschaulicht werden:

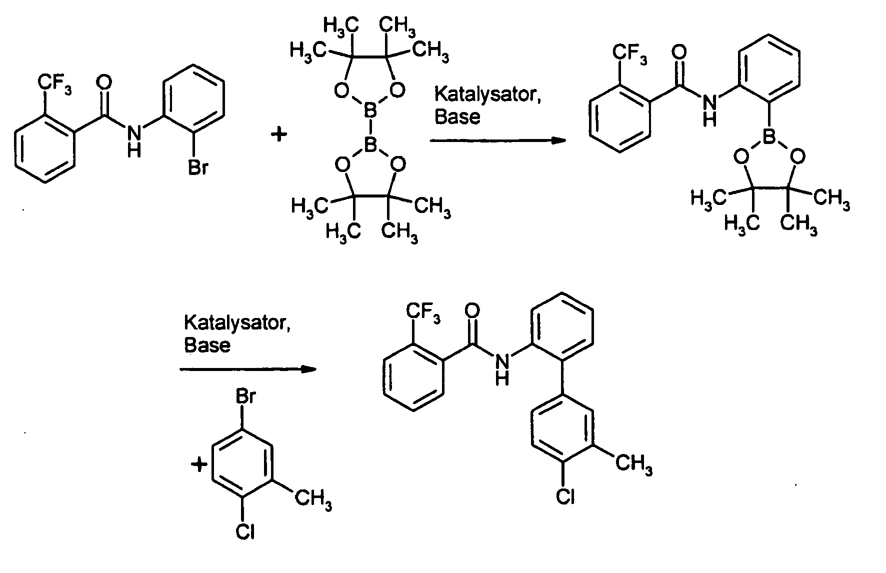

Die zur Durchführung des erfindungsgemäßen Verfahrens D) als Ausgangsstoffe benötigten Halogenbenzamide der Formel (IV) sind bereits weiter oben im Zusammenhang mit dem erfindungsgemäßen Verfahren B) beschrieben worden.

Die zur Durchführung des erfindungsgemäßen Verfahrens D) weiterhin als Ausgangsstoffe benötigten Diboran-Derivate sind durch die Formel (VIII) allgemein definiert. In dieser Formel (VIII) stehen G⁵ und G⁶ bevorzugt jeweils für Methyl, Ethyl, Propyl, Butyl oder gemeinsam fürTetramethylethylen.

Die Diboran-Derivate der Formel (VIII) sind allgemein bekannte Synthesechemikalien.

Die zur Durchführung des erfindungsgemäßen Verfahrens D) weiterhin als Ausgangsstoffe benötigten Halogenbenzolderivaten der Formel (VII) sind bereits weiter oben im Zusammenhang mit dem erfindungsgemäßen Verfahren C) beschrieben worden.

Als Verdünnungsmittel zur Durchführung der erfindungsgemäßen Verfahrens A), E) und F) kommen alle inerten organischen Lösungsmittel in Betracht. Hierzu gehören vorzugsweise aliphatische, alicyclische oder aromatische Kohlenwasserstoffe, wie beispielsweise Petrolether, Hexan, Heptan, Cyclohexan, Methylcyclohexan, Benzol, Toluol, Xylol oder Decalin; halogenierte Kohlenwasserstoffe, wie beispielsweise Chlorbenzol, Dichlorbenzol, Dichlormethan, Chloroform, Tetrachlormethan, Dichlorethan oder Trichlorethan; Ether, wie Diethylether, Diisopropylether, Methyl-t-butylether, Methyl-t-Amylether, Dioxan, Tetrahydrofuran, 1,2-Dimethoxyethan, 1,2-Diethoxyethan oder Anisol oder Amide, wie N,N-Dimethylformamid, N,N-Dimethylacetamid, N-Methylformanilid, N-Methylpyrrolidon oder Hexamethylphosphorsäuretriamid.

Die erfindungsgemäßen Verfahren A), E) und F) werden gegebenenfalls in Gegenwart eines geeigneten Säureakzeptors durchgeführt. Als solche kommen alle üblichen anorganischen oder organischen Basen infrage. Hierzu gehören vorzugsweise Erdalkalimetall- oder Alkalimetallhydride, -hydroxide, -amide, -alkoholate, -acetate, -carbonate oder -hydrogencarbonate, wie beispielsweise Natriumhydrid, Natriumamid, Natrium-methylat, Natrium-ethylat, Kalium-tert.-butylat, Natriumhydroxid, Kaliumhydroxid, Ammoniumhydroxid, Natriumacetat, Kaliumacetat, Calciumacetat, Ammoniumacetat, Natriumcarbonat, Kaliumcarbonat, Kaliumhydrogencarbonat, Natriumhydrogencarbonat oder Caesiumcarbonat, sowie tertiäre Amine, wie Trimethylamin, Triethylamin, Tributylamin, N,N-Dimethylanilin, N,N-Dimethyl-benzylamin, Pyridin, N-Methylpiperidin, N-Methylmorpholin, N,N-Dimethylaminopyridin, Diazabicyclooctan (DABCO), Diazabicyclononen (DBN) oder Diazabicycloundecen (DBU).

Die Reaktionstemperaturen können bei der Durchführung der erfindungsgemäßen Verfahren A), E) und F) in einem größeren Bereich variiert werden. Im allgemeinen arbeitet man bei Temperaturen von 0°C bis 150°C, vorzugsweise bei Temperaturen von 20°C bis 110°C.

Zur Durchführung des erfindungsgemäßen Verfahrens A) zur Herstellung der Verbindungen der Formel (I) setzt man pro Mol des Benzoylhalogenides der Formel (II) im allgemeinen 0,2 bis 5 Mol, vorzugsweise 0,5 bis 2 Mol an Anilinderivat der Formel (III) ein. Die Aufarbeitung erfolgt nach üblichen Methoden.

Zur Durchführung des erfindungsgemäßen Verfahrens E) zur Herstellung der Verbindungen der Formel (III) setzt man pro Mol des Benzoylhalogenides der Formel (II) im allgemeinen 0,2 bis 5 Mol, vorzugsweise 0,5 bis 2 Mol an 2-Bromanilin oder 2-Iodanilin ein. Die Aufarbeitung erfolgt nach üblichen Methoden.

Zur Durchführung des erfindungsgemäßen Verfahrens F) zur Herstellung der Verbindungen der Formel (VI) setzt man pro Mol des Benzoylhalogenides der Formel (II) im allgemeinen 0,2 bis 5 Mol, vorzugsweise 0,5 bis 2 Mol an Anilinboronsäurederivat der Formel (IX) ein. Die Aufarbeitung erfolgt nach üblichen Methoden.

Als Verdünnungsmittel zur Durchführung der erfindungsgemäßen Verfahren B), C) und D) kommen alle inerten organischen Lösungsmittel in Betracht. Hierzu gehören vorzugsweise aliphatische, alicyclische oder aromatische Kohlenwasserstoffe, wie beispielsweise Petrolether, Hexan, Heptan, Cyclohexan, Methylcyclohexan, Benzol, Toluol, Xylol oder Decalin; Ether, wie Diethylether, Diisopropylether, Methyl-t-butylether, Methyl-t-Amylether, Dioxan, Tetrahydrofuran, 1,2-Dimethoxyethan, 1,2-Diethoxyethan oder Anisol; Nitrile, wie Acetonitril, Propionitril, n- oder i-Butyronitril oder Benzonitril; Amide, wie N,N-Dimethylformamid, N,N-Dimethylacetamid, N-Methylformanilid, N-Methylpyrrolidon oder Hexamethylphosphorsäuretriamid; Ester wie Essigsäuremethylester oder Essigsäureethylester; Sulfoxide, wie Dimethylsulfoxid; Sulfone, wie Sulfolan; Alkohole, wie Methanol, Ethanol, n- oder i-Propanol, n-, i-, sek- oder tert-Butanol, Ethandiol, Propan-1,2-diol, Ethoxyethanol, Methoxyethanol, Diethylenglykolmonomethylether, Diethylenglykolmonoethylether, deren Gemische mit Wasser oder reines Wasser.

Die Reaktionstemperaturen können bei der Durchführung der erfindungsgemäßen Verfahrens B), C) und D) in einem größeren Bereich variiert werden. Im allgemeinen arbeitet man bei Temperaturen von 0°C bis 150°C, vorzugsweise bei Temperaturen von 20°C bis 110°C.

Die erfindungsgemäßen Verfahren B), C) und D) werden gegebenenfalls in Gegenwart eines geeigneten Säureakzeptors durchgeführt. Als solche kommen alle üblichen anorganischen oder organischen Basen infrage. Hierzu gehören vorzugsweise Erdalkalimetall- oder Alkalimetallhydride, -hydroxide, -amide, -alkoholate, -acetate, fluoride, phosphate, -carbonate oder -hydrogencarbonate, wie beispielsweise Natriumhydrid, Natriumamid, Lithiumdiisopropylamid, Natrium-methylat, Natrium-ethylat, Kalium-tert.-butylat, Natriumhydroxid, Kaliumhydroxid, Natriumacetat, Natriumphosphat, Kaliumphosphat, Kaliumfluorid, Cäsiumfluorid, Natriumcarbonat, Kaliumcarbonat, Kaliumhydrogencarbonat, Natriumhydrogencarbonat oder Cäsiumcarbonat, sowie tertiäre Amine, wie Trimethylamin, Triethylamin, Tributylamin, N,N-Dimethylanilin, N,N-Dimethyl-benzylamin, Pyridin, N-Methylpiperidin, N-Methylmorpholin, N,N-Dimethylaminopyridin, Diazabicyclooctan (DABCO), Diazabicyclononen (DBN) oder Diazabicycloundecen (DBU).

Die erfindungsgemäßen Verfahren B), C) und D) werden in Gegenwart eines Katalysators, wie beispielsweise eines Palladiumsalzes oder -komplexes, durchgeführt. Hierzu kommen vorzugsweise Palladiumchlorid, Palladiumacetat, Tetrakis-(triphenylphosphin)-Palladium, Bis-(triphenylphosphin)-Palladiumdichlorid oder (1,1'-Bis-(diphenylphosphino)ferrocenpalladium(II)chlorid) infrage.

Es kann auch ein Palladiumkomplex in der Reaktionsmischung erzeugt werden, wenn man ein Palladiumsalz und ein Komplexligand, wie z.B. Triethylphosphan, Tri-tert-butylphosphan, Tricyclohexylphosphan, 2-(Dicyclohexylphosphan)-biphenyl, 2-(Di-tert-butylphosphan)-biphenyl, 2-(Dicyclohexylphosphan)-2'-(N,N-dimethylamino)-biphenyl, Triphenylphosphan, Tris-(o-tolyl)-phosphan, Natrium-3-(Diphenylphosphino)benzolsulfonat, Tris-2-(methoxyphenyl)-phosphan, 2,2'-Bis-(diphenylphosphan)-1,1'-binaphthyl, 1,4-Bis-(diphenylphosphan)-butan, 1,2-Bis-(diphenylphosphan)-ethan, 1,4-Bis-(dicyclohexylphosphan)-butan, 1,2-Bis-(dicyclohexylphosphan)-ethan, 2-(Dicyclohexylphosphan)-2'-(N,N-dimethylamino)-biphenyl, Bis(diphenylphosphino)ferrocen oder Tris-(2,4-tert-butylphenyl)-phosphit getrennt zur Reaktion zugibt.

Zur Durchführung des erfindungsgemäßen Verfahrens B) zur Herstellung der Verbindungen der Formel (I) setzt man pro Mol des Halogenbenzamids der Formel (IV) im allgemeinen 1 bis 15 Mol, vorzugsweise 2 bis 8 Mol an Boronsäurederivat der Formel (V) ein. Die Aufarbeitung erfolgt nach üblichen Methoden.

Zur Durchführung des erfindungsgemäßen Verfahrens C) zur Herstellung der Verbindungen der Formel (I) setzt man pro Mol des Benzamid-Boronsäure-Derivates der Formel (VI) im allgemeinen 1 bis 15 Mol, vorzugsweise 2 bis 8 Mol an Halogenbenzolderivat der Formel (VII) ein. Die Aufarbeitung erfolgt nach üblichen Methoden.

Zur Durchführung des erfindungsgemäßen Verfahrens D) zur Herstellung der Verbindungen der Formel (I) setzt man pro Mol des Halogenbenzamid der Formel (IV) im allgemeinen 1 bis 15 Mol, vorzugsweise 1 bis 5 Mol an Diboran-Derivat der Formel (VIII) und 1 bis 15 Mol, vorzugsweise 1 bis 5 Mol an Halogenbenzolderivat der Formel (VII) ein.

Die erfindungsgemäßen Verfahren A), B), C), D), E) und F) werden im allgemeinen unter Normaldruck durchgeführt. Es ist jedoch auch möglich, unter erhöhtem oder vermindertem Druck - im allgemeinen zwischen 0,1 bar und 10 bar - zu arbeiten.
Die erfindungsgemäßen Wirkstoffe der Formel (I) weisen eine starke mikrobizide Wirkung auf und können zur Bekämpfung von unerwünschten Mikroorganismen, wie Fungi und Bakterien, im Pflanzenschutz und im Materialschutz eingesetzt werden

Fungizide lassen sich im Pflanzenschutz zur Bekämpfung von Plasmodiophoromycetes, Oomycetes, Chytridiomycetes, Zygomycetes, Ascomycetes, Basidiomycetes und Deuteromycetes einsetzen.

Bakterizide lassen sich im Pflanzenschutz zur Bekämpfung von Pseudomonadaceae, Rhizobiaceae, Enterobacteriaceae, Corynebacteriaceae und Streptomycetaceae einsetzen.

Beispielhaft aber nicht begrenzend seien einige Erreger von pilzlichen und bakteriellen Erkrankungen, die unter die oben aufgezählten Oberbegriffe fallen, genannt:
Xanthomonas-Arten, wie beispielsweise Xanthomonas campestris pv. oryzae;
Pseudomonas-Arten, wie beispielsweise Pseudomonas syringae pv. lachrymans;
Erwinia-Arten, wie beispielsweise Erwinia amylovora;
Pythium-Arten, wie beispielsweise Pythium ultimum;
Phytophthora-Arten, wie beispielsweise Phytophthora infestans;
Pseudoperonospora-Arten, wie beispielsweise Pseudoperonospora humuli oder Pseudoperonospora cubensis;
Plasmopara-Arten, wie beispielsweise Plasmopara viticola;
Bremia-Arten, wie beispielsweise Bremia lactucae;
Peronospora-Arten, wie beispielsweise Peronospora pisi oder P. brassicae;
Erysiphe-Arten, wie beispielsweise Erysiphe graminis;
Sphaerotheca-Arten, wie beispielsweise Sphaerotheca fuliginea;
Podosphaera-Arten, wie beispielsweise Podosphaera leucotricha;
Venturia-Arten, wie beispielsweise Venturia inaequalis;
Pyrenophora-Arten, wie beispielsweise Pyrenophora teres oder P. graminea (Konidienform: Drechslera, Syn: Helminthosporium);
Cochliobolus-Arten, wie beispielsweise Cochliobolus sativus (Konidienform: Drechslera, Syn: Helminthosporium);
Uromyces-Arten, wie beispielsweise Uromyces appendiculatus;
Puccinia-Arten, wie beispielsweise Puccinia recondita;
Sclerotinia-Arten, wie beispielsweise Sclerotinia sclerotiorum;
Tilletia-Arten, wie beispielsweise Tilletia caries;
Ustilago-Arten, wie beispielsweise Ustilago nuda oder Ustilago avenae;
Pellicularia-Arten, wie beispielsweise Pellicularia sasakii;
Pyricularia-Arten, wie beispielsweise Pyricularia oryzae;
Fusarium-Arten, wie beispielsweise Fusarium culmorum;
Botrytis-Arten, wie beispielsweise Botrytis cinerea;
Uncinula-Arten, wie beispielsweise Uncinula necator;
Septoria-Arten, wie beispielsweise Septoria nodorum;
Leptosphaeria-Arten, wie beispielsweise Leptosphaeria nodorum;
Cercospora-Arten, wie beispielsweise Cercospora canescens;
Alternaria-Arten, wie beispielsweise Altemaria brassicae;
Pseudocercosporella-Arten, wie beispielsweise Pseudocercosporella herpotrichoides.

Die erfindungsgemäßen Wirkstoffe der Formel (I) weisen auch eine starke stärkende Wirkung in Pflanzen auf. Sie eignen sich daher zur Mobilisierung pflanzeneigener Abwehrkräfte gegen Befall durch unerwünschte Mikroorganismen.

Unter pflanzenstärkenden (resistenzinduzierenden) Stoffen sind im vorliegenden Zusammenhang solche Substanzen zu verstehen, die in der Lage sind, das Abwehrsystem von Pflanzen so zu stimulieren, dass die behandelten Pflanzen bei nachfolgender Inokulation mit unerwünschten Mikroorganismen weitgehende Resistenz gegen diese Mikroorganismen entfalten.

Unter unerwünschten Mikroorganismen sind im vorliegenden Fall phytopathogene Pilze, Bakterien und Viren zu verstehen. Die erfindungsgemäßen Stoffe können also eingesetzt werden, um Pflanzen innerhalb eines gewissen Zeitraumes nach der Behandlung gegen den Befall durch die genannten Schaderreger zu schützen. Der Zeitraum, innerhalb dessen Schutz herbeigeführt wird, erstreckt sich im allgemeinen von 1 bis 10 Tage, vorzugsweise 1 bis 7 Tage nach der Behandlung der Pflanzen mit den Wirkstoffen.
Die gute Pflanzenverträglichkeit der Wirkstoffe in den zur Bekämpfung von Pflanzenkrankheiten notwendigen Konzentrationen erlaubt eine Behandlung von oberirdischen Pflanzenteilen, von Pflanz- und Saatgut, und des Bodens.

Die erfindungsgemäßen Wirkstoffe der Formel (I) eignen sich auch zur Steigerung des Ernteertrages. Sie sind außerdem mindertoxisch und weisen eine gute Pflanzenverträglichkeit auf

Die erfindungsgemäßen Wirkstoffe der Formel (I) können gegebenenfalls in bestimmten Konzentrationen und Aufwandmengen auch als Herbizide, zur Beeinflussung des Pflanzenwachstums, sowie zur Bekämpfung von tierischen Schädlingen verwendet werden. Sie lassen sich gegebenenfalls auch als Zwischen- und Vorprodukte für die Synthese weiterer Wirkstoffe einsetzen.

Erfindungsgemäß können alle Pflanzen und Pflanzenteile behandelt werden. Unter Pflanzen werden hierbei alle Pflanzen und Pflanzenpopulationen verstanden, wie erwünschte und unerwünschte Wildpflanzen oder Kulturpflanzen (einschließlich natürlich vorkommender Kulturpflanzen). Kulturpflanzen können Pflanzen sein, die durch konventionelle Züchtungs- und Optimierungsmethoden oder durch biotechnologische und gentechnologische Methoden oder Kombinationen dieser Methoden erhalten werden können, einschließlich der transgenen Pflanzen und einschließlich der durch Sortenschutzrechte schützbaren oder nicht schützbaren Pflanzensorten. Unter Pflanzenteilen sollen alle oberirdischen und unterirdischen Teile und Organe der Pflanzen, wie Spross, Blatt, Blüte und Wurzel verstanden werden, wobei beispielhaft Blätter, Nadeln, Stängel, Stämme, Blüten, Fruchtkörper, Früchte und Samen sowie Wurzeln, Knollen und Rhizome aufgeführt werden. Zu den Pflanzenteilen gehört auch Erntegut sowie vegetatives und generatives Vermehrungsmaterial, beispielsweise Stecklinge, Knollen, Rhizome, Ableger und Samen.

Die erfindungsgemäße Behandlung der Pflanzen und Pflanzenteile mit den Wirkstoffen der Formel (I) erfolgt direkt oder durch Einwirkung auf deren Umgebung, Lebensraum oder Lagerraum nach den üblichen Behandlungsmethoden, z.B. durch Tauchen, Sprühen, Verdampfen, Vernebeln, Streuen, Aufstreichen und bei Vermehrungsmaterial, insbesondere bei Samen, weiterhin durch ein- oder mehrschichtiges Umhüllen.

Im Materialschutz lassen sich die erfindungsgemäßen Stoffe der Formel (I) zum Schutz von technischen Materialien gegen Befall und Zerstörung durch unerwünschte Mikroorganismen einsetzen.

Unter technischen Materialien sind im vorliegenden Zusammenhang nichtlebende Materialien zu verstehen, die für die Verwendung in der Technik zubereitet worden sind. Beispielsweise können technische Materialien, die durch erfindungsgemäße Wirkstoffe vor mikrobieller Veränderung oder Zerstörung geschützt werden sollen, Klebstoffe, Leime, Papier und Karton, Textilien, Leder, Holz, Anstrichmittel und Kunststoffartikel, Kühlschmierstoffe und andere Materialien sein, die von Mikroorganismen befallen oder zersetzt werden können. Im Rahmen der zu schützenden Materialien seien auch Teile von Produktionsanlagen, beispielsweise Kühlwasserkreisläufe, genannt, die durch Vermehrung von Mikroorganismen beeinträchtigt werden können. Im Rahmen der vorliegenden Erfindung seien als technische Materialien vorzugsweise Klebstoffe, Leime, Papiere und Kartone, Leder, Holz, Anstrichmittel, Kühlschmiermittel und Wärmeübertragungsflüssigkeiten genannt, besonders bevorzugt Holz.

Als Mikroorganismen, die einen Abbau oder eine Veränderung der technischen Materialien bewirken können, seien beispielsweise Bakterien, Pilze, Hefen, Algen und Schleimorganismen genannt. Vorzugsweise wirken die erfindungsgemäßen Wirkstoffe gegen Pilze, insbesondere Schimmelpilze, holzverfärbende und holzzerstörende Pilze (Basidiomyceten) sowie gegen Schleimorganismen und Algen.

Es seien beispielsweise Mikroorganismen der folgenden Gattungen genannt:
Altemaria, wie Alternaria tenuis,
Aspergillus, wie Aspergillus niger,
Chaetomium, wie Chaetomium globosum,
Coniophora, wie Coniophora puetana,
Lentinus, wie Lentinus tigrinus,
Penicillium, wie Penicillium glaucum,
Polyporus, wie Polyporus versicolor,
Aureobasidium, wie Aureobasidium pullulans,
Sclerophoma, wie Sclerophoma pityophila,
Trichoderma, wie Trichoderma viride,
Escherichia, wie Escherichia coli,
Pseudomonas, wie Pseudomonas aeruginosa,
Staphylococcus, wie Staphylococcus aureus.

Die Wirkstoffe können in Abhängigkeit von ihren jeweiligen physikalischen und/oder chemischen Eigenschaften in die üblichen Formulierungen überführt werden, wie Lösungen, Emulsionen, Suspensionen, Pulver, Schäume, Pasten, Granulate, Aerosole, Feinstverkapselungen in polymeren Stoffen und in Hüllmassen für Saatgut, sowie ULV-Kalt- und Warmnebel-Formulierungen.

Diese Formulierungen werden in bekannter Weise hergestellt, z.B. durch Vermischen der Wirkstoffe mit Streckmitteln, also flüssigen Lösungsmitteln, unter Druck stehenden verflüssigten Gasen und/oder festen Trägerstoffen, gegebenenfalls unter Verwendung von oberflächenaktiven Mitteln, also Emulgiermitteln und/oder Dispergiermitteln und/oder schaumerzeugenden Mitteln. Im Falle der Benutzung von Wasser als Streckmittel können z.B. auch organische Lösungsmittel als Hilfslösungsmittel verwendet werden. Als flüssige Lösungsmittel kommen im wesentlichen in Frage: Aromaten, wie Xylol, Toluol oder Alkylnaphthaline, chlorierte Aromaten oder chlorierte aliphatische Kohlenwasserstoffe, wie Chlorbenzole, Chlorethylene oder Methylenchlorid, aliphatische Kohlenwasserstoffe, wie Cyclohexan oder Paraffine, z.B. Erdölfraktionen, Alkohole, wie Butanol oder Glycol sowie deren Ether und Ester, Ketone, wie Aceton, Methylethylketon, Methylisobutylketon oder Cyclohexanon, stark polare Lösungsmittel, wie Dimethylformamid und Dimethylsulfoxid, sowie Wasser. Mit verflüssigten gasförmigen Streckmitteln oder Trägerstoffen sind solche Flüssigkeiten gemeint, welche bei normaler Temperatur und unter Normaldruck gasförmig sind, z.B. Aerosol-Treibgase, wie Halogenkohlenwasserstoffe sowie Butan, Propan, Stickstoff und Kohlendioxid.

Als feste Trägerstoffe kommen in Frage: z.B. natürliche Gesteinsmehle, wie Kaoline, Tonerden, Talkum, Kreide, Quarz, Attapulgit, Montmorillonit oder Diatomeenerde und synthetische Gesteinsmehle, wie hochdisperse Kieselsäure, Aluminiumoxid und Silikate. Als feste Trägerstoffe für Granulate kommen in Frage: z.B. gebrochene und fraktionierte natürliche Gesteine wie Calcit, Marmor, Bims, Sepiolith, Dolomit sowie synthetische Granulate aus anorganischen und organischen Mehlen sowie Granulate aus organischem Material wie Sägemehl, Kokosnussschalen, Maiskolben und Tabakstängel. Als Emulgier und/oder schaumerzeugende Mittel kommen in Frage: z.B. nichtionogene und anionische Emulgatoren, wie Polyoxyethylen-Fettsäureester, Polyoxyethylen-Fettalkoholether, z.B. Alkylarylpolyglycolether, Alkylsulfonate, Alkylsulfate, Arylsulfonate sowie Eiweißhydrolysate. Als Dispergiermittel kommen in Frage: z.B. Lignin-Sulfitablaugen und Methylcellulose.

Es können in den Formulierungen Haftmittel wie Carboxymethylcellulose, natürliche und synthetische pulverige, körnige oder latexförmige Polymere verwendet werden, wie Gummiarabicum, Polyvinylalkohol, Polyvinylacetat, sowie natürliche Phospholipide, wie Kephaline und Lecithine, und synthetische Phospholipide. Weitere Additive können mineralische und vegetabile Öle sein.

Es können Farbstoffe wie anorganische Pigmente, z.B. Eisenoxid, Titanoxid, Ferrocyanblau und organische Farbstoffe, wie Alizarin-, Azo- und Metallphthalocyaninfarbstoffe und Spurennährstoffe, wie Salze von Eisen, Mangan, Bor, Kupfer, Kobalt, Molybdän und Zink verwendet werden.

Die Formulierungen enthalten im allgemeinen zwischen 0,1 und 95 Gewichtsprozent Wirkstoff, vorzugsweise zwischen 0,5 und 90 %.

Die erfindungsgemäßen Wirkstoffe können als solche oder in ihren Formulierungen auch in Mischung mit bekannten Fungiziden, Bakteriziden, Akariziden, Nematiziden oder Insektiziden verwendet werden, um so z.B. das Wirkungsspektrum zu verbreitern oder Resistenzentwicklungen vorzubeugen. In vielen Fällen erhält man dabei synergistische Effekte, d.h. die Wirksamkeit der Mischung ist größer als die Wirksamkeit der Einzelkomponenten.

Als Mischpartner kommen zum Beispiel folgende Verbindungen in Frage:
**Fungizide:**
   2-Phenylphenol; 8-Hydroxyquinoline sulfate;
   Acibenzolar-S-methyl; Aldimorph; Amidoflumet; Ampropylfos; Ampropylfos-potassium; Andoprim; Anilazine; Azaconazole; Azoxystrobin;
   Benalaxyl; Benodanil; Benomyl; Benthiavalicarb-isopropyl; Benzamacril; Benzamacril-isobutyl; Bilanafos; Binapacryl; Biphenyl; Bitertanol; Blasticidin-S; Bromuconazole; Bupirimate; Buthiobate; Butylamine;
   Calcium polysulfide; Capsimycin; Captafol; Captan; Carbendazim; Carboxin; Carpropamid; Carvone; Chinomethionat; Chlobenthiazone; Chlorfenazole; Chloroneb; Chlorothalonil; Chlozolinate; Clozylacon; Cyazofamid; Cyflufenamid; Cymoxanil; Cyproconazole; Cyprodinil; Cyprofuram;
   Dagger G; Debacarb; Dichlofluanid; Dichlone; Dichlorophen; Diclocymet; Diclomezine; Dicloran; Diethofencarb; Difenoconazole; Diflumetorim; Dimethirimol; Dimethomorph; Dimoxystrobin; Diniconazole; Diniconazole-M; Dinocap; Diphenylamine; Dipyrithione; Ditalimfos; Dithianon; Dodine; Drazoxolon;
   Edifenphos; Epoxiconazole; Ethaboxam; Ethirimol; Etridiazole;
   Famoxadone; Fenamidone; Fenapanil; Fenarimol; Fenbuconazole; Fenfuram; Fenhexamid; Fenitropan; Fenoxanil; Fenpiclonil; Fenpropidin; Fenpropimorph; Ferbam; Fluazinam; Flubenzimine; Fludioxonil; Flumetover; Flumorph; Fluoromide; Fluoxastrobin; Fluquinconazole; Flurprimidol; Flusilazole; Flusulfamide; Flutolanil; Flutriafol; Folpet; Fosetyl-Al; Fosetyl-sodium; Fuberidazole; Furalaxyl; Furametpyr; Furcarbanil; Furmecyclox;
   Guazatine; Hexachlorobenzene; Hexaconazole; Hymexazol;
   Imazalil; Imibenconazole; Iminoctadine triacetate; Iminoctadine tris(albesil; Iodocarb; Ipconazole; Iprobenfos; Iprodione; Iprovalicarb; Irumamycin; Isoprothiolane; Isovaledione;
   Kasugamycin; Kresoxim-methyl;
   Mancozeb; Maneb; Meferimzone; Mepanipyrim; Mepronil; Metalaxyl; Metalaxyl-M; Metconazole; Methasulfocarb; Methfuroxam; Metiram; Metominostrobin; Metsulfovax; Mildiomycin; Myclobutanil; Myclozolin;
   Natamycin; Nicobifen; Nitrothal-isopropyl; Noviflumuron; Nuarimol;
   Ofurace; Orysastrobin; Oxadixyl; Oxolinic acid; Oxpoconazole; Oxycarboxin; Oxyfenthiin;
   Paclobutrazol; Pefurazoate; Penconazole; Pencycuron; Phosdiphen; Phthalide; Picoxystrobin; Piperalin; Polyoxins; Polyoxorim; Probenazole; Prochloraz; Procymidone; Propamocarb; Propanosine-sodium; Propiconazole; Propineb; Proquinazid; Prothioconazole; Pyraclostrobin; Pyrazophos; Pyrifenox; Pyrimethanil; Pyroquilon; Pyroxyfur; Pyrrolnitrine;
   Quinconazole; Quinoxyfen; Quintozene; Simeconazole; Spiroxamine; Sulfur;
   Tebuconazole; Tecloftalam; Tecnazene; Tetcyclacis; Tetraconazole; Thiabendazole; Thicyofen; Thifluzamide; Thiophanate-methyl; Thiram; Tioxymid; Tolclofos-methyl; Tolylfluanid; Triadimefon; Triadimenol; Triazbutil; Triazoxide; Tricyclamide; Tricyclazole; Tridemorph; Trifloxystrobin; Triflumizole; Triforine; Triticonazole;
   Uniconazole; Validamycin A; Vinclozolin; Zineb; Ziram; Zoxamide; (2S)-N-[2-[4-[[3-(4-chlorophenyl)-2-propynyl]oxy]-3-methoxyphenyl]ethyl]-3-methyl- 2-[(methylsulfonyl)amino]-butanamide; 1-(1-naphthalenyl)-1H-pyrrole-2,5-dione; 2,3,5,6-tetrachloro-4-(methylsulfonyl)-pyridine; 2-amino-4-methyl-N-phenyl-5-thiazolecarboxamide; 2-chloro-N-(2,3-dihydro-1,1,3-trimethyl-1H-inden-4-yl)-3-pyridincarboxam ide; 3,4,5-trichloro-2,6-pyridinedicarbonitrile; Actinovate; cis-1-(4-chlorophenyl)-2-(1H-1,2,4-triazole-1-yl)-cycloheptanol; methyl 1-(2,3-dihydro-2,2-dimethyl-1H-inden-1-yl)-1H-imidazole-5-carboxylate; monopotassium carbonate; N-(6-methoxy-3-pyridinyl)-cyclopropanecarboxamide; N-butyl-8-(1,1-dimethylethyl)-1-oxaspiro[4.5]decan-3-amine; Sodium tetrathiocarbonate;
   sowie Kupfersalze und -zubereitungen, wie Bordeaux mixture; Copper hydroxide; Copper naphthenate; Copper oxychloride; Copper sulfate; Cufraneb; Cuprous oxide; Mancopper; Oxine-copper.
**Bakterizide:**
   Bronopol, Dichlorophen, Nitrapyrin, Nickel-dimethyldithiocarbamat, Kasugamycin, Octhilinon, Furancarbonsäure, Oxytetracyclin, Probenazol, Streptomycin, Tecloftalam, Kupfersulfat und andere Kupfer-Zubereitungen.
**Insektizide / Akarizide / Nematizide:**
   Abamectin, ABG-9008, Acephate, Acequinocyl, Acetamiprid, Acetoprole, Acrinathrin, AKD-1022, AKD-3059, AKD-3088, Alanycarb, Aldicarb, Aldoxycarb, Allethrin, Allethrin 1R-isomers, Alpha-Cypermethrin (Alphamethrin), Amidoflumet, Aminocarb, Amitraz, Avermectin, AZ-60541, Azadirachtin, Azamethiphos, Azinphos-methyl, Azinphos-ethyl, Azocyclotin,
   Bacillus popilliae, Bacillus sphaericus, Bacillus subtilis, Bacillus thuringiensis, Bacillus thuringiensis strain EG-2348, Bacillus thuringiensis strain GC-91, Bacillus thuringiensis strain NCTC-11821, Baculoviren, Beauveria bassiana, Beauveria tenella, Bendiocarb, Benfuracarb, Bensultap, Benzoximate, Beta-Cyfluthrin, Beta-Cypermethrin, Bifenazate, Bifenthrin, Binapacryl, Bioallethrin, Bioallethrin-S-cyclopentyl-isomer, Bioethanomethrin, Biopermethrin, Bioresmethrin, Bistrifluron, BPMC, Brofenprox, Bromophos-ethyl, Bromopropylate, Bromfenvinfos (-methyl), BTG-504, BTG-505, Bufencarb, Buprofezin, Butathiofos, Butocarboxim, Butoxycarboxim, Butylpyridaben,
   Cadusafos, Camphechlor, Carbaryl, Carbofuran, Carbophenothion, Carbosulfan, Cartap, CGA-50439, Chinomethionat, Chlordane, Chlordimeform, Chloethocarb, Chlorethoxyfos, Chlorfenapyr, Chlorfenvinphos, Chlorfluazuron, Chlormephos, Chlorobenzilate, Chloropicrin, Chlorproxyfen, Chlorpyrifos-methyl, Chlorpyrifos (-ethyl), Chlovaporthrin, Chromafenozide, Cis-Cypermethrin, Cis-Resmethrin, Cis-Permethrin, Clocythrin, Cloethocarb, Clofentezine, Clothianidin, Clothiazoben, Codlemone, Coumaphos, Cyanofenphos, Cyanophos, Cycloprene, Cycloprothrin, Cydia pomonella, Cyfluthrin, Cyhalothrin, Cyhexatin, Cypermethrin, Cyphenothrin (1R-trans-isomer), Cyromazine,
   DDT, Deltamethrin, Demeton-S-methyl, Demeton-S-methylsulphon, Diafenthiuron, Dialifos, Diazinon, Dichlofenthion, Dichlorvos, Dicofol, Dicrotophos, Dicyclanil, Diflubenzuron, Dimethoate, Dimethylvinphos, Dinobuton, Dinocap, Dinotefuran, Diofenolan, Disulfoton, Docusat-sodium, Dofenapyn, DOWCO-439,
   Eflusilanate, Emamectin, Emamectin-benzoate, Empenthrin (1R-isomer), Endosulfan, Entomopthora spp., EPN, Esfenvalerate, Ethiofencarb, Ethiprole, Ethion, Ethoprophos, Etofenprox, Etoxazole, Etrimfos,
   Famphur, Fenamiphos, Fenazaquin, Fenbutatin oxide, Fenfluthrin, Fenitrothion, Fenobucarb, Fenothiocarb, Fenoxacrim, Fenoxycarb; Fenpropathrin, Fenpyrad, Fenpyrithrin, Fenpyroximate, Fensulfothion, Fenthion, Fentrifanil, Fenvalerate, Fipronil, Flonicamid, Fluacrypyrim, Fluazuron, Flubenzimine, Flubrocythrinate, Flucycloxuron, Flucythrinate, Flufenerim, Flufenoxuron, Flufenprox, Flumethrin, Flupyrazofos, Flutenzin (Flufenzine), Fluvalinate, Fonofos, Formetanate, Formothion, Fosmethilan, Fosthiazate, Fubfenprox (Fluproxyfen), Furathiocarb,
   Gamma-HCH, Gossyplure, Grandlure, Granuloseviren,
   Halfenprox, Halofenozide, HCH, HCN-801, Heptenophos, Hexaflumuron, Hexythiazox, Hydramethylnone, Hydroprene,
   IKA-2002, Imidacloprid, Imiprothrin, Indoxacarb, Iodofenphos, Iprobenfos, Isazofos, Isofenphos, Isoprocarb, Isoxathion, Ivermectin,
   Japonilure, Kadethrin, Kernpolyederviren, Kinoprene,
   Lambda-Cyhalothrin, Lindane, Lufenuron,
   Malathion, Mecarbam, Mesulfenfos, Metaldehyd, Metam-sodium, Methacrifos, Methamidophos, Metharhizium anisopliae, Metharhizium flavoviride, Methidathion, Methiocarb, Methomyl, Methoprene, Methoxychlor, Methoxyfenozide, Metolcarb, Metoxadiazone, Mevinphos, Milbemectin, Milbemycin, MKI-245, MON-45700, Monocrotophos, Moxidectin, MTI-800,
   Naled, NC-104, NC-170, NC-184, NC-194, NC-196, Niclosamide, Nicotine, Nitenpyram, Nithiazine, NNI-0001, NNI-0101, NNI-0250, NNI-9768, Novaluron, Noviflumuron,
   OK-5101, OK-5201, OK-9601, OK-9602, OK-9701, OK-9802, Omethoate, Oxamyl, Oxydemeton-methyl,
   Paecilomyces fumosoroseus, Parathion-methyl, Parathion (-ethyl), Permethrin (cis-, trans-), Petroleum, PH-6045, Phenothrin (IR-trans isomer), Phenthoate, Phorate, Phosalone, Phosmet, Phosphamidon, Phosphocarb, Phoxim, Piperonyl butoxide, Pirimicarb, Pirimiphos-methyl, Pirimiphos-ethyl, Prallethrin, Profenofos, Promecarb, Propaphos, Propargite, Propetamphos, Propoxur, Prothiofos, Prothoate, Protrifenbute, Pymetrozine, Pyraclofos, Pyresmethrin, Pyrethrum, Pyridaben, Pyridalyl, Pyridaphenthion, Pyridathion, Pyrimidifen, Pyriproxyfen,
   Quinalphos, Resmethrin, RH-5849, Ribavirin, RU-12457, RU-15525,
   S-421, S-1833, Salithion, Sebufos, SI-0009, Silafluofen, Spinosad, Spirodiclofen, Spiromesifen, Sulfluramid, Sulfotep, Sulprofos, SZI-121,
   Tau-Fluvalinate, Tebufenozide, Tebufenpyrad, Tebupirimfos, Teflubenzuron, Tefluthrin, Temephos, Temivinphos, Terbam, Terbufos, Tetrachlorvinphos, Tetradifon, Tetramethrin, Tetramethrin (1R-isomer), Tetrasul, Theta-Cypermethrin, Thiacloprid, Thiamethoxam, Thiapronil, Thiatriphos, Thiocyclam hydrogen oxalate, Thiodicarb, Thiofanox, Thiometon, Thiosultap-sodium, Thuringiensin, Tolfenpyrad, Tralocythrin, Tralomethrin, Transfluthrin, Triarathene, Triazamate, Triazophos, Triazuron, Trichlophenidine, Trichlorfon, Triflumuron, Trimethacarb,
   Vamidothion, Vaniliprole, Verbutin, Verticillium lecanii,
   WL-108477, WL-40027, YI-5201, YI-5301, YI-5302, XMC, Xylylcarb,
   ZA-3274, Zeta-Cypermethrin, Zolaprofos, ZXI-8901,
   die Verbindung 3-Methyl-phenyl-propylcarbamat (Tsumacide Z),
   die Verbindung 3-(5-Chlor-3-pyridinyl)-8-(2,2,2-trifluorethyl)-8-azabicyclo[3.2.1]-octan-3-carbonitril (CAS-Reg.-Nr. 185982-80-3) und das entsprechende 3-endo-Isomere (CAS-Reg.-Nr. 185984-60-5) (vgl. WO-96/37494, WO-98/25923),
sowie Präparate, welche insektizid wirksame Pflanzenextrakte, Nematoden, Pilze oder Viren enthalten.

Auch eine Mischung mit anderen bekannten Wirkstoffen, wie Herbiziden oder mit Düngemitteln und Wachstumsregulatoren, Safener bzw. Semiochemicals ist möglich.

Darüber hinaus weisen die erfindungsgemäßen Verbindungen der Formel (I) auch sehr gute antimykotische Wirkungen auf. Sie besitzen ein sehr breites antimykotisches Wirkungsspektrum, insbesondere gegen Dermatophyten und Sprosspilze, Schimmel und diphasische Pilze ( z.B. gegen Candida-Spezies wie Candida albicans, Candida glabrata) sowie Epidermophyton floccosum, Aspergillus-Spezies wie Aspergillus niger und Aspergillus fumigatus, Trichophyton-Spezies wie Trichophyton mentagrophytes, Microsporon-Spezies wie Microsporon canis und audouinii. Die Aufzählung dieser Pilze stellt keinesfalls eine Beschränkung des erfassbaren mykotischen Spektrums dar, sondern hat nur erläuternden Charakter.

Die Wirkstoffe können als solche, in Form ihrer Formulierungen oder den daraus bereiteten Anwendungsformen, wie gebrauchsfertige Lösungen, Suspensionen, Spritzpulver, Pasten, lösliche Pulver, Stäubemittel und Granulate angewendet werden. Die Anwendung geschieht in üblicher Weise, z.B. durch Gießen, Verspritzen, Versprühen, Verstreuen, Verstäuben, Verschäumen, Bestreichen usw. Es ist ferner möglich, die Wirkstoffe nach dem Ultra-Low-Volume-Verfahren auszubringen oder die Wirkstoffzubereitung oder den Wirkstoff selbst in den Boden zu injizieren. Es kann auch das Saatgut der Pflanzen behandelt werden.

Beim Einsatz der erfindungsgemäßen Wirkstoffe als Fungizide können die Aufwandmengen je nach Applikationsart innerhalb eines größeren Bereiches variiert werden. Bei der Behandlung von Pflanzenteilen liegen die Aufwandmengen an Wirkstoff im allgemeinen zwischen 0,1 und 10.000 g/ha, vorzugsweise zwischen 10 und 1.000 g/ha. Bei der Saatgutbehandlung liegen die Aufwandmengen an Wirkstoff im allgemeinen zwischen 0,001 und 50 g pro Kilogramm Saatgut, vorzugsweise zwischen 0,01 und 10 g pro Kilogramm Saatgut. Bei der Behandlung des Bodens liegen die Aufwandmengen an Wirkstoff im allgemeinen zwischen 0,1 und 10.000 g/ha, vorzugsweise zwischen 1 und 5.000 g/ha.

Wie bereits oben erwähnt, können erfindungsgemäß alle Pflanzen und deren Teile behandelt werden. In einer bevorzugten Ausführungsform werden wild vorkommende oder durch konventionelle biologische Zuchtmethoden, wie Kreuzung oder Protoplastenfusion erhaltenen Pflanzenarten und Pflanzensorten sowie deren Teile behandelt. In einer weiteren bevorzugten Ausführungsform werden transgene Pflanzen und Pflanzensorten, die durch gentechnologische Methoden gegebenenfalls in Kombination mit konventionellen Methoden erhalten wurden (Genetic Modified Organisms) und deren Teile behandelt. Der Begriff "Teile" bzw. "Teile von Pflanzen" oder "Pflanzenteile" wurde oben erläutert.

Besonders bevorzugt werden erfindungsgemäß Pflanzen der jeweils handelsüblichen oder in Gebrauch befindlichen Pflanzensorten behandelt. Unter Pflanzensorten versteht man Pflanzen mit neuen Eigenschaften ("Traits"), die sowohl durch konventionelle Züchtung, durch Mutagenese oder durch rekombinante DNA-Techniken gezüchtet worden sind. Dies können Sorten, Rassen, Bio- und Genotypen sein.

Je nach Pflanzenarten bzw. Pflanzensorten, deren Standort und Wachstumsbedingungen (Böden, Klima, Vegetationsperiode, Ernährung) können durch die erfindungsgemäße Behandlung auch überadditive ("synergistische") Effekte auftreten. So sind beispielsweise erniedrigte Aufwandmengen und/oder Erweiterungen des Wirkungsspektrums und/oder eine Verstärkung der Wirkung der erfindungsgemäß verwendbaren Stoffe und Mittel, besseres Pflanzenwachstum, erhöhte Toleranz gegenüber hohen oder niedrigen Temperaturen, erhöhte Toleranz gegen Trockenheit oder gegen Wasser- bzw. Bodensalzgehalt, erhöhte Blühleistung, erleichterte Ernte, Beschleunigung der Reife, höhere Ernteerträge, höhere Qualität und/oder höherer Ernährungswert der Ernteprodukte, höhere Lagerfähigkeit und/oder Bearbeitbarkeit der Ernteprodukte möglich, die über die eigentlich zu erwartenden Effekte hinausgehen.

Zu den bevorzugten erfindungsgemäß zu behandelnden transgenen (gentechnologisch erhaltenen) Pflanzen bzw. Pflanzensorten gehören alle Pflanzen, die durch die gentechnologische Modifikation genetisches Material erhielten, welches diesen Pflanzen besondere vorteilhafte wertvolle Eigenschaften ("Traits") verleiht. Beispiele für solche Eigenschaften sind besseres Pflanzenwachstum, erhöhte Toleranz gegenüber hohen oder niedrigen Temperaturen, erhöhte Toleranz gegen Trockenheit oder gegen Wasser- bzw. Bodensalzgehalt, erhöhte Blühleistung, erleichterte Ernte, Beschleunigung der Reife, höhere Ernteerträge, höhere Qualität und/oder höherer Ernährungswert der Ernteprodukte, höhere Lagerfähigkeit und/oder Bearbeitbarkeit der Ernteprodukte. Weitere und besonders hervorgehobene Beispiele für solche Eigenschaften sind eine erhöhte Abwehr der Pflanzen gegen tierische und mikrobielle Schädlinge, wie gegenüber Insekten, Milben, pflanzenpathogenen Pilzen, Bakterien und/oder Viren sowie eine erhöhte Toleranz der Pflanzen gegen bestimmte herbizide Wirkstoffe. Als Beispiele transgener Pflanzen werden die wichtigen Kulturpflanzen, wie Getreide (Weizen, Reis), Mais, Soja, Kartoffel, Baumwolle, Raps sowie Obstpflanzen (mit den Früchten Äpfel, Birnen, Zitrusfrüchten und Weintrauben) erwähnt, wobei Mais, Soja, Kartoffel, Baumwolle und Raps besonders hervorgehoben werden. Als Eigenschaften ("Traits") werden besonders hervorgehoben die erhöhte Abwehr der Pflanzen gegen Insekten durch in den Pflanzen entstehende Toxine, insbesondere solche, die durch das genetische Material aus Bacillus Thuringiensis (z.B. durch die Gene CryIA(a), CryIA(b), CryIA(c), CryIIA, CryIIIA, CryIIIB2, Cry9c Cry2Ab, Cry3Bb und CryIF sowie deren Kombinationen) in den Pflanzen erzeugt werden (im folgenden "Bt Pflanzen"). Als Eigenschaften ("Traits") werden auch besonders hervorgehoben die erhöhte Abwehr von Pflanzen gegen Pilze, Bakterien und Viren durch Systemische Akquirierte Resistenz (SAR), Systemin, Phytoalexine, Elicitoren sowie Resistenzgene und entsprechend exprimierte Proteine und Toxine. Als Eigenschaften ("Traits") werden weiterhin besonders hervorgehoben die erhöhte Toleranz der Pflanzen gegenüber bestimmten herbiziden Wirkstoffen, beispielsweise Imidazolinonen, Sulfonylharnstoffen, Glyphosate oder Phosphinotricin (z.B. "PAT"-Gen). Die jeweils die gewünschten Eigenschaften ("Traits") verleihenden Gene können auch in Kombinationen miteinander in den transgenen Pflanzen vorkommen. Als Beispiele für "Bt Pflanzen" seien Maissorten, Baumwollsorten, Sojasorten und Kartoffelsorten genannt, die unter den Handelsbezeichnungen YIELD GARD^{®} (z.B. Mais, Baumwolle, Soja), KnockOut^{®} (z.B. Mais), StarLink^{®} (z.B. Mais), Bollgard^{®} (Baumwolle), Nucoton^{®} (Baumwolle) und NewLeaf^{®} (Kartoffel) vertrieben werden. Als Beispiele für Herbizid tolerante Pflanzen seien Maissorten, Baumwollsorten und Sojasorten genannt, die unter den Handelsbezeichnungen Roundup Ready^{®} (Toleranz gegen Glyphosate z.B. Mais, Baumwolle, Soja), Liberty Link^{®} (Toleranz gegen Phosphinotricin, z.B. Raps), IMI^{®} (Toleranz gegen Imidazolinone) und STS^{®} (Toleranz gegen Sulfonylharnstoffe z.B. Mais) vertrieben werden. Als Herbizid resistente (konventionell auf Herbizid-Toleranz gezüchtete) Pflanzen seien auch die unter der Bezeichnung Clearfield^{®} vertriebenen Sorten (z.B. Mais) erwähnt. Selbstverständlich gelten diese Aussagen auch für in der Zukunft entwickelte bzw. zukünftig auf den Markt kommende Pflanzensorten mit diesen oder zukünftig entwickelten genetischen Eigenschaften ("Traits").

Die aufgeführten Pflanzen können besonders vorteilhaft erfindungsgemäß mit den Verbindungen der allgemeinen Formeln (I), bzw. den erfindungsgemäßen Wirkstoffmischungen behandelt werden. Die bei den Wirkstoffen bzw. Mischungen oben angegebenen Vorzugsbereiche gelten auch für die Behandlung dieser Pflanzen. Besonders hervorgehoben sei die Pflanzenbehandlung mit den im vorliegenden Text speziell aufgeführten Verbindungen bzw. Mischungen.

Die Herstellung und die Verwendung der erfindungsgemäßen Stoffe geht aus den folgenden Beispielen hervor.

### Herstellungsbeispiele

### Beispiel 1

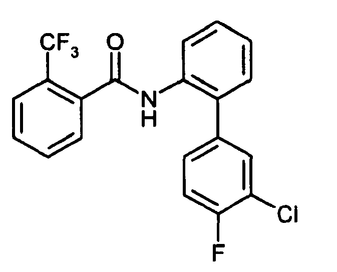

### Verfahren A):

0,288 g (1,3 mmol) 3'-Chlor-4'-fluor-1,1'-biphenyl-2-amin werden in 3 ml Tetrahydrofuran gelöst, mit 0,36 ml (2,6 mmol) Triethylamin und 0,25 g (1,56 mmol) 2-Trifluormethylbenzoesäurechlorid (gelöst in 3 ml Tetrahydrofuran) versetzt. Die Reaktionslösung wird für 16 h bei 60°C gerührt. Zur Aufarbeitung wird aufkonzentriert und das Rohprodukt mittels Säulenchromatographie (Cyclohexan/Essigsäureethylester 2:1) gereinigt.

Man erhält 0,491 g (96 % der Theorie) *N*-(3'-Chlor-4'-fluor-1,1'-biphenyl-2-yl)2-(trifluormethyl)benzamid (Verbindung 35, vgl. Tabelle 1) mit dem logP (pH2,3) = 3,81.

### Beispiel 2

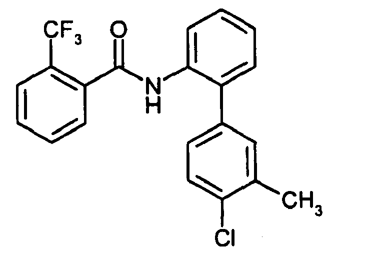

### Verfahren D)

226 mg (1,1 mmol) 2-Chlor-5-bromtoluol werden mit 245 mg (2,5 mmol) Kaliumacetat und 279 mg (1,1 mmol) Pinacoldiboronester in 8 ml Dimethylformamid (möglichst sauerstofffrei) gelöst und mit einer katalytischen Menge (0,1 eq.) PdCl₂(dppf) versetzt. Die Reaktionsmischung wird 2 h bei 80-90°C gerührt und nach Abkühlen mit 5 eq. 2 M Natriumcarbonat-Lösung, 344 mg (1,0 mmol) des *N*-(2-Bromphenyl)-2-(trifluormethyl)benzamid (gelöst in 4 ml Dimethylformamid) und weiteren 0,1 eq. Katalysator versetzt. Die Reaktionslösung wird für 16 h bei 80-90°C gerührt. Zur Aufarbeitung wird mit 2 ml Wasser und 8 ml Essigsäureethylester versetzt. Die organische Phase wird eingeengt und mittels Säulenchromatographie (Cyclohexan/Essigsäureethylester 1:1) gereinigt.

Man erhält 151 mg (39 % der Theorie) *N*-(4'-Chlor-3'-methyl-1,1'-biphenyl-2-yl)-2-(trifluormethyl)benzamid (Verbindung 42, vgl. Tabelle 1) mit dem logP (pH2,3) = 4,18.

Analog den Beispielen 1 und 2 sowie entsprechend den allgemeinen Beschreibungen der erfindungsgemäßen Verfahren A) bis D) können die in der folgenden Tabelle 1 genannten Verbindungen der Formel (I) erhalten werden. In der letzten Spalte der Tabelle ist angegeben, zu welcher erfindungsgemäßen Gruppe neuer Verbindungen die jeweilige Substanz gehört.

**Tabelle 1**

| | | | | | | |
|---|---|---|---|---|---|---|
| 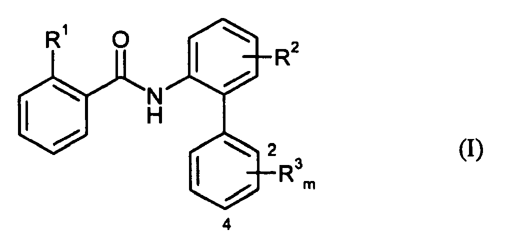 | | | | | | |

| **Nr.** | **R¹** | **R²** | **m** | **R³** | **logP (pH2,3)** | **Gruppe** |
|---|---|---|---|---|---|---|
| 1 | CF₃ | H | 1 2 | 2,4-Cl₂ | 4,00 | 1 |
| 2 | CF₃ | H | 2 | 2-CH₃, 4-Cl | 4,14 | 1 |
| 3 | CF₃ | H | 2 | 3,4-Cl₂ | 4,13 | 1 |
| 4 | CF₃ | H | 2 | 3,4-F₂ | 3,55 | 1 |
| 5 | CF₃ | H | 2 | 3-F, 4-Cl | 3,76 | 1 |
| 6 | CF₃ | H | 2 | 3-Cl, 4-F | 3,81 | 1 |
| 7 | CF₃ | H | 2 | 2,4-F₂ | 3,41 | 1 |
| 8 | CF₃ | H | 2 | 3-F, 4-OCF₃ | 4,08 | 1 |
| 9 | CF₃ | H | 2 | 3-CF₃,4-Cl | 4,18 | 1 |
| 10 | CF₃ | H | 2 | 3-CF₃,4-CH₃ | 4,18 | 1 |
| 11 | CF₃ | H | 2 | 3-CF₃, 4-OCF₃ | 4,41 | 1 |
| 12 | CF₃ | H | 2 | 3-CF₃, 4-F | 3,90 | 1 |
| 13 | CF₃ | H | 2 | 3-CH₃, 4-Cl | 4,18 | 1 |
| 14 | CF₃ | H | 2 | 3,5-Cl₂ | 4,16 | 1 |
| 15 | I | H | 2 | 3,4-Cl₂ | 4,06 | 1 |
| 16 | CF₃ | H | 2 | 2-F, 4-Cl | 3,68 | 1 |
| 17 | CF₃ | 5'-F | 2 | 3,4-Cl₂ | 4,11 | 1 |
| 18 | CF₃ | 3'-F | 2 | 3,4-Cl₂ | 3,81 | 1 |

### Herstellung von Ausgangsstoffen der Formel (III)

### Beispiel (III-1)

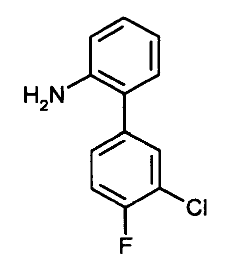

Unter Argonatmosphäre werden 38,8 g (223 mmol) der 3-Chlor-4-fluorphenylboronsäure, 40,6 g (186 mmol) 2-Iodanilin in 220 ml Toluol, 22 ml Ethanol und 45 ml einer 4 M Natriumhydrogencarbonat-Lösung gelöst. 4,3 g (4 mmol) Tetrakis(triphenylphosphin)palladium(0) werden zugegeben und die Reaktionslösung 2-16 h auf 80°C erhitzt. Zur Aufarbeitung werden die Phasen getrennt, die organische Phase über Magnesiumsulfat getrocknet und aufkonzentriert. Die Reinigung des Rohproduktes erfolgt durch Säulenchromatographie (Cyclohexan/Essigsäureethylester 3:1 ) und/oder durch Umkristallisation.

Man erhält 19,8 g (4 8% der Theorie) an 3'-Chlor-4'-fluor-1,1'-biphenyl-2-amin mit dem logP (pH2,3) = 3,01.

### Herstellung von Ausgangsstoffen der Formel (IV)

### Beispiel (IV-1)

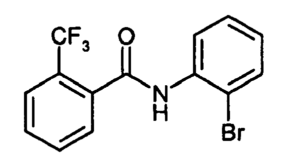

7,5 g (0,044 mol) 2-Bromanilin werden in 100 ml Acetonitril vorgelegt und nacheinander mit 7,8 g (0,057 mol) Kaliumcarbonat und 10,0 g (0,048 mol) 2-Trifluormethylbenzoesäurechlorid versetzt. Die Reaktionslösung wird für 16 h unter Rückfluss erhitzt. Zur Aufarbeitung wird aufkonzentriert und mit Cyclohexan/Essigsäureethylester an Kieselgel chromatographiert.

Man erhält 9,75 g (65 % der Theorie) *N*-(2-Bromphenyl)-2-(trifluormethyl)benzamid mit dem logP (pH2,3) = 2,99.

Die Bestimmung der in den voranstehenden Tabellen und Herstellungsbeispielen angegebenen logP-Werte erfolgt gemäß EEC-Directive 79/831 Annex V.48 durch HPLC (High Performance Liquid Chromatography) an einer Phasenumkehrsäule (C18). Temperatur: 43°C.

Die Bestimmung erfolgt im saurem Bereich bei pH 2.3 mit 0,1 % wässriger Phosphorsäure und Acetonitril als Eluenten; linearer Gradient von 10 % Acetonitril bis 90 % Acetonitril.

Die Eichung erfolgt mit unverzweigten Alkan-4-onen (mit 3 bis 16 Kohlenstoffatomen), deren logP-Werte bekannt sind (Bestimmung der logP-Werte anhand der Retentionszeit durch lineare Interpolation zwischen zwei aufeinanderfolgenden Alkanonen).

### Anwendungsbeispiete

### Beispiel A

### Podosphaera-Test (Apfel) / protektiv

| | |
|---|---|
| Lösungsmittel: | 24,5 Gewichtsteile Aceton |
| | 24,5 Gewichtsteile Dimethylacetamid |
| Emulgator: | 1,0 Gewichtsteile Alkyl-Aryl-Polyglykolether |

Zur Herstellung einer zweckmäßigen Wirkstoffzubereitung vermischt man 1 Gewichtsteil Wirkstoff mit den angegebenen Mengen Lösungsmittel und Emulgator und verdünnt das Konzentrat mit Wasser auf die gewünschte Konzentration.

Zur Prüfung auf protektive Wirksamkeit werden junge Pflanzen mit der Wirkstoffzubereitung in der angegebenen Aufwandmenge besprüht. Nach Antrocknen des Spritzbelages werden die Pflanzen mit einer wässrigen Sporensuspension des Apfelmehltauerregers *Podosphaera leucotricha* inokuliert. Die Pflanzen werden dann im Gewächshaus bei ca. 23°C und einer relativen Luftfeuchtigkeit von ca. 70 % aufgestellt.

10 Tage nach der Inokulation erfolgt die Auswertung. Dabei bedeutet 0 % ein Wirkungsgrad, der demjenigen der Kontrolle entspricht, während ein Wirkungsgrad von 100 % bedeutet, dass kein Befall beobachtet wird.

Wirkstoffe, Aufwandmengen und Versuchsergebnisse gehen aus der folgenden Tabelle hervor.

**Tabelle A**

| **Podosphaera-Test (Apfel) / protektiv** | | | |
|---|---|---|---|
| Wirkstoff | | Aufwandmenge an Wirkstoff in g/ha | Wirkungsgrad in % |
| 4 | 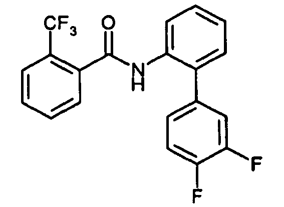 | 100 | 100 |
| 5 | 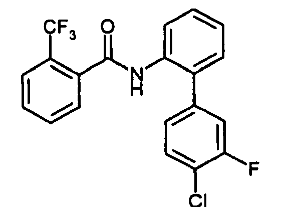 | 100 | 100 |
| 6 | 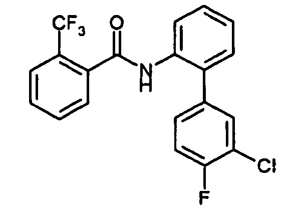 | 100 | 100 |
| 3 | 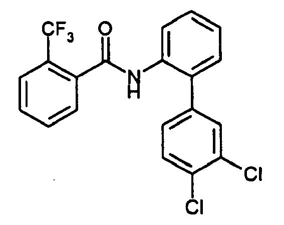 | 100 | 100 |
| 12 | 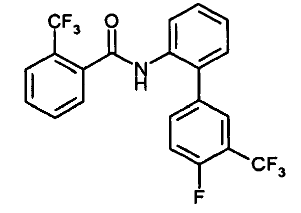 | 100 | 94 |
| 13 | 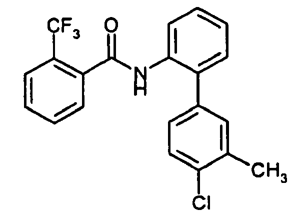 | 100 | 100 |
| 1 | 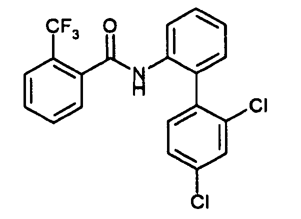 | 100 | 100 |
| 2 | 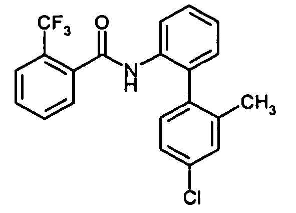 | 100 | 99 |

### Beispiel B

### Sphaerotheca-Test (Gurke) / protektiv

| | |
|---|---|
| Lösungsmittel: | 24,5 Gewichtsteile Aceton |
| | 24,5 Gewichtsteile Dimethylacetamid |
| Emulgator: | 1,0 Gewichtsteile Alkyl-Aryl-Polyglykolether |

Zur Herstellung einer zweckmäßigen Wirkstoffzubereitung vermischt man 1 Gewichtsteil Wirkstoff mit den angegebenen Mengen Lösungsmittel und Emulgator und verdünnt das Konzentrat mit Wasser auf die gewünschte Konzentration.

Zur Prüfung auf protektive Wirksamkeit werden junge Pflanzen mit der Wirkstoffzubereitung in der angegebenen Aufwandmenge besprüht. Nach Antrocknen des Spritzbelages werden die Pflanzen mit einer wässrigen Sporensuspension von *Sphaerotheca fuliginea* inokuliert. Die Pflanzen werden dann bei ca. 23°C und einer relativen Luftfeuchtigkeit von ca. 70 % im Gewächshaus aufgestellt.

7 Tage nach der Inokulation erfolgt die Auswertung. Dabei bedeutet 0 % ein Wirkungsgrad, der demjenigen der Kontrolle entspricht, während ein Wirkungsgrad von 100 % bedeutet, dass kein Befall beobachtet wird.

Wirkstoffe, Aufwandmengen und Versuchsergebnisse gehen aus der folgenden Tabelle hervor.

**Tabelle B**

| **Sphaerotheca-Test (Gurke) / protektiv** | | | |
|---|---|---|---|
| Wirkstoff | | Aufwandmenge an Wirkstoff in g/ha | Wirkungsgrad in % |
| 4 | 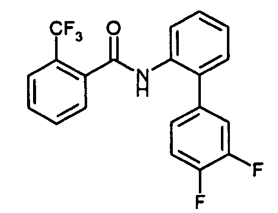 | 100 | 82 |
| 5 | 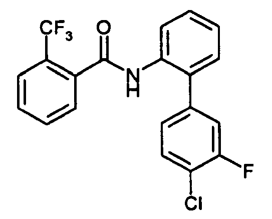 | 100 | 95 |
| 6 | 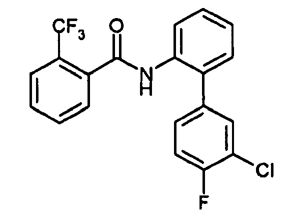 | 100 | 93 |
| 3 | 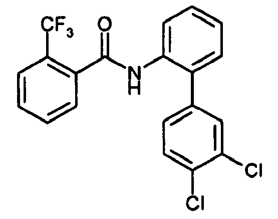 | 100 | 100 |
| 12 | 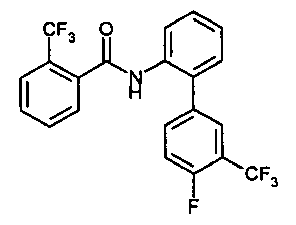 | 100 | 100 |
| 13 | 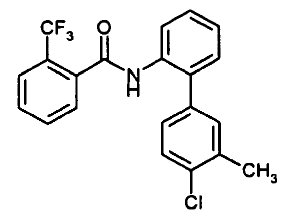 | 100 | 100 |
| 1 | 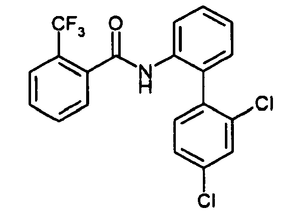 | 100 | 94 |
| 2 | 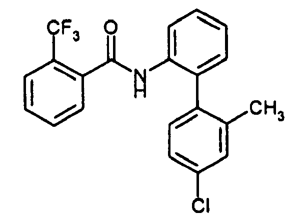 | 100 | 100 |

### Beispiel C

### Venturia - Test (Apfel) / protektiv

| | |
|---|---|
| Lösungsmittel: | 24,5 Gewichtsteile Aceton |
| | 24,5 Gewichtsteile Dimethylacetamid |
| Emulgator: | 1,0 Gewichtsteile Alkyl-Aryl-Polyglykolether |

Zur Herstellung einer zweckmäßigen Wirkstoffzubereitung vermischt man 1 Gewichtsteil Wirkstoff mit den angegebenen Mengen Lösungsmittel und Emulgator und verdünnt das Konzentrat mit Wasser auf die gewünschte Konzentration.

Zur Prüfung auf protektive Wirksamkeit werden junge Pflanzen mit der Wirkstoffzubereitung in der angegebenen Aufwandmenge besprüht. Nach Antrocknen des Spritzbelages werden die Pflanzen mit einer wässrigen Konidiensuspension des Apfelschorferregers *Venturia inaequalis* inokuliert und verbleiben dann 1 Tag bei ca. 20°C und 100 % relativer Luftfeuchtigkeit in einer Inkubationskabine.

Die Pflanzen werden dann im Gewächshaus bei ca. 21°C und einer relativen Luftfeuchtigkeit von ca. 90 % aufgestellt.

10 Tage nach der Inokulation erfolgt die Auswertung. Dabei bedeutet 0 % ein Wirkungsgrad, der demjenigen der Kontrolle entspricht, während ein Wirkungsgrad von 100 % bedeutet, dass kein Befall beobachtet wird.

Wirkstoffe, Aufwandmengen und Versuchsergebnisse gehen aus der folgenden Tabelle hervor.

**Tabelle C**

| **Venturia - Test (Apfel) / protektiv** | | | |
|---|---|---|---|
| Wirkstoff | | Aufwandmenge an Wirkstoff in g/ha | Wirkungsgrad in % |
| 4 | 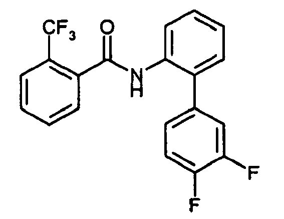 | 100 | 100 |
| 5 | 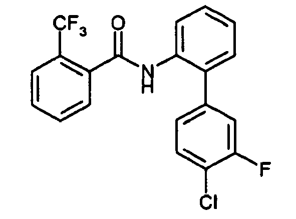 | 100 | 100 |
| 6 | 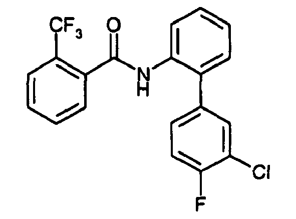 | 100 | 100 |
| 3 | 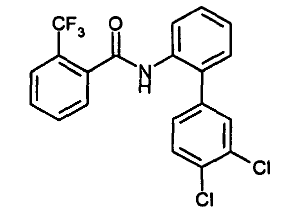 | 100 | 100 |
| 13 | 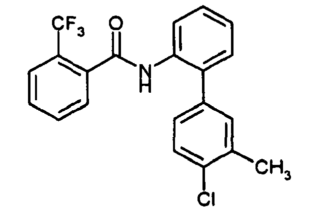 | 100 | 100 |
| 2 | 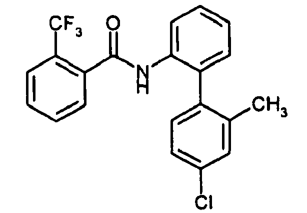 | 100 | 100 |

### Beispiel D

### Pyrenophora teres-Test (Gerste) / protektiv

| | |
|---|---|
| Lösungsmittel: | 25 Gewichtsteile N,N-Dimethylacetamid |
| Emulgator: | 0,6 Gewichtsteile Alkylarylpolyglykolether |

Zur Herstellung einer zweckmäßigen Wirkstoffzubereitung vermischt man 1 Gewichtsteil Wirkstoff mit den angegebenen Mengen Lösungsmittel und Emulgator und verdünnt das Konzentrat mit Wasser auf die gewünschte Konzentration.

Zur Prüfung auf protektive Wirksamkeit werden junge Pflanzen mit der Wirkstoffzubereitung in der angegeben Aufwandmenge besprüht. Nach Antrocknen des Spritzbelages werden die Pflanzen mit einer Konidiensuspension von *Pyrenophora teres* besprüht. Die Pflanzen verbleiben 48 Stunden bei 20°C und 100 % relativer Luftfeuchtigkeit in einer Inkubationskabine.

Die Pflanzen werden dann in einem Gewächshaus bei einer Temperatur von ca. 20°C und relativen Luftfeuchtigkeit von ca. 80 % aufgestellt.

Wirkstoffe, Aufwandmengen und Versuchsergebnisse gehen aus der folgenden Tabelle hervor.

**Tabelle D**

| **Pyrenophora teres-Test (Gerste) / protektiv** | | | |
|---|---|---|---|
| Wirkstoff | | Aufwandmenge an Wirkstoff in g/ha | Wirkungsgrad in % |
| 3 | 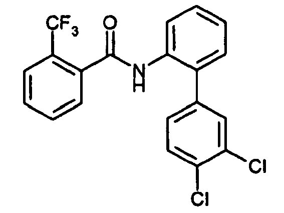 | 500 | 100 |

## Patentansprüche

1. Biphenylbenzamid-Derivate der Formel (Ia) 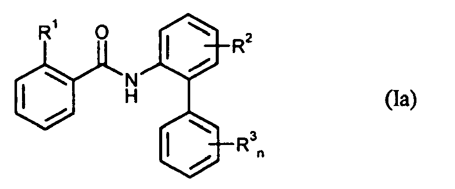 in welcher
R¹ für Methyl, Trifluormethyl, Chlor, Brom oder Iod steht,
R² für Wasserstoff oder Fluor steht,
R³ für Halogen, Cyano, Nitro, C₁-C₆-Alkyl, C₁-C₄-Alkoxy, C₁-C₄-Alkylthio, C₁-C₄-Alkylsulfonyl, C₂-C₆-A!kenyt, C₃-C₆-Cycloalkyl, oder für C₁-C₆-Halogenalkyl, C₁-C₆-Halogenalkoxy, C₁-C₆-Halogenalkylthio oder C₁-C₆-Halogenalkylsulfonyl mit jeweils 1 bis 13 Halogenatomen steht,
n für 2, 3, 4 oder 5 steht, wobei die Reste R³ gleich oder verschieden sein können.

2. Biphenylbenzamid-Derivate der Formel (Ia) gemäß Anspruch 1, in welcher
R¹ für Trifluormethyl!, Chlor, Brom oder Iod steht,
R² für Wasserstoff oder Fluor steht,
R³ für Halogen, C₁-C₄-Alkyl, C₁-C₄-Alkoxy, C₁-C₄-Alkylthio, C₂-C₄-Alkenyl, Cyclopropyl, Cyclobutyl, Cyclopentyl, Cyclohexyl, oder für C₁-C₄-Halogenalkyl, C₁-C₄-Halogenalkoxy oder C₁-C₄-Halogenalkylthio mit jeweils 1 bis 9 Halogenatomen steht,
n für 2, 3 steht, wobei die Reste R³ gleich oder verschieden sein können.

3. Mittel zum Bekämpfen unerwünschter Mikroorganismen, **gekennzeichnet durch** einen Gehalt an mindestens einem Biphenylbenzamid-Derivat der Formeln (Ia) gemäß Anspruch 1, neben Streckmitteln und/oder oberflächenaktiven Stoffen.

4. Verwendung von Biphenylbenzamid-Derivaten der Formeln (Ia) gemäß Anspruch 1 zum Bekämpfen unerwünschter Mikroorganismen.

5. Verfahren zum Bekämpfen unerwünschter Mikroorganismen, **dadurch gekennzeichnet, dass** man Biphenylbenzamid-Derivate der Formel (Ia) gemäß Anspruch 1 auf die Mikroorganismen und/oder deren Lebensraum ausbringt.

6. Verfahren zum Herstellen von Mitteln zum Bekämpfen unerwünschter Mikroorganismen, **dadurch gekennzeichnet, dass** man Biphenylbenzamid-Derivate der Formeln (Ia) gemäß Anspruch 1 mit Streckmitteln und/oder oberflächenaktiven Stoffen vermischt.

## Claims

1. Biphenylbenzamide derivatives of the formula (Ia) 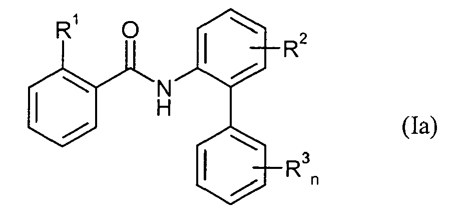 in which
R¹ represents methyl, trifluoromethyl, chlorine, bromine or iodine,
R² represents hydrogen or fluorine,
R³ represents halogen, cyano, nitro, C₁-C₆-alkyl, C₁-C₄-alkoxy, C₁-C₄-alkylthio, C₁-C₄-alkylsulphonyl, C₂-C₆-alkenyl, C₃-C₆-cycloalkyl, or represents C₁-C₆-haloalkyl, C₁-C₆-haloalkoxy, C₁-C₆-haloalkylthio or C₁-C₆-haloalkylsulphonyl having in each case 1 to 13 halogen atoms,
n represents 2, 3, 4, or 5, where the radicals R³ may be identical or different.

2. Biphenylbenzamide derivatives of the formula (Ia) according to Claim 1 in which
R¹ represents trifluoromethyl, chlorine, bromine or iodine,
R² represents hydrogen or fluorine,
R³ represents halogen, C₁-C₄-alkyl, C₁-C₄-alkoxy, C₁-C₄-alkylthio, C₂-C₄-alkenyl, cyclopropyl, cyclobutyl, cyclopentyl, cyclohexyl, or represents C₁-C₄-haloalkyl, C₁-C₄-haloalkoxy or C₁-C₄-haloalkylthio having in each case 1 to 9 halogen atoms,
n represents 2, 3, where the radicals R³ may be identical or different.

3. Compositions for controlling unwanted microorganisms, **characterized in that** they comprise at least one biphenylbenzamide derivative of the formula (Ia) according to Claim 1 in addition to extenders and/or surfactants.

4. Use of biphenylbenzamide derivatives of the formulae (Ia) according to Claim 1 for controlling unwanted microorganisms.

5. Method for controlling unwanted microorganisms, **characterized in that** biphenylbenzamide derivatives of the formula (Ia) according to Claim 1 are applied to the microorganisms and/or their habitat.

6. Process for preparing compositions for controlling unwanted microorganisms, **characterized in that** biphenylbenzamide derivatives of the formula (Ia) according to Claim 1 are mixed with extenders and/or surfactants.

## Revendications

1. Dérivés de biphénylbenzamide de la formule (Ia) 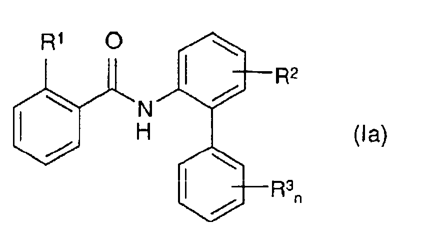 dans laquelle
R¹ représente un groupe méthyle, trifluorométhyle, chlore, brome ou Iode,
R² représente l'hydrogène ou le fluor,
R³ représente un halogène, un groupe cyano, nitro, alkyle en C₁-C₆, alcoxy, en C₁-C₄, alkylthio en C₁-C₄, alkylsulfonyle en C₁-C₄, alcényle en C₂-C₆, cycloalkyle en C₃-C₆ ou halogénoalkyle en C₁-C₆, halogénoalcoxy en C₁-C₆, halogénoalkylthlo en C₁-C₆ ou halogénoalkylsulfonyle en C₁-C₆ avec à chaque fols de 1 à 13 atomes d'halogène,
n représente 2, 3, 4 ou 5, les restes R³ pouvant être identiques ou différents.

2. Dérivés de biphénylbenzamide de la formule (Ia) selon la revendication 1, dans lesquels
R¹ représente un groupe trifluorométhyle, chlore, brome ou iode,
R² représente l'hydrogène ou le fluor,
R³ représente un atome d'halogène, un groupe alkyle en C₁-C₄, alcoxy en C₁-C₄, alkylthio en C₁-C₄, alcényle en C₂-C₄, cyclopropyle, cyclobutyle, cyclopentyle, cyclohexyle ou halogénoalkyle en C₁-C₄, halogénoalcoxy en C₁-C₄ ou halogénoalkylthio en C₁-C₄ avec à chaque fois de 1 à 9 atomes d'halogène,
n représente 2, 3, les restes R³ pouvant être identiques ou différents.

3. Agent pour combattre des micro-organismes indésirables, **caractérisé par** une teneur en au moins un dérivé de biphénylbenzamide de la formule (Ia) selon la revendication 1 en plus de diluants et/ou de matières tensio-actives.

4. Utilisation de dérivés de biphénylbenzamide de la formule (Ia) selon la revendication 1 pour combattre des micro-organismes indésirables.

5. Procédé pour combattre des micro-organismes indésirables, **caractérisé en ce que** l'on applique des dérivés de biphénylbenzamide de la formule (Ia) selon la revendication 1 sur les micro-organismes et/ou leur biotope.

6. Procédé pour la préparation d'agents pour combattre des micro-organismes indésirables, **caractérisé en ce que** l'on mélange des dérivés de biphénylbenzamide de la formule (Ia) selon la revendication 1 avec des diluants et/ou des matières tensio-actives.
